# FASCICULE DE BREVET EUROPEEN

(11) **EP 0 979 079 B1**
(45) Date de publication et mention de la délivrance du brevet: **16.06.2004**
(21) Numéro de dépôt: 98921552.0
(22) Date de dépôt: 17.04.1998
(51) Int. Cl.: A61K 31/44

(54) **UTILISATION DE DERIVES DE LA TETRAHYDROPYRIDINE POUR LA PREPARATION DE MEDICAMENTS POUR LE TRAITEMENT DES MALADIES ENTRAINANT UNE DEMYELINISATION**
VERWENDUNG VON TETRAHYDROPYRIDINDERIVATEN ZUR HERSTELLUNG VON ARZNEIMITTELN ZUR BEHANDLUNG DEMYELINISIERENDER ERKRANKUNGEN
USE OF TETRAHYDROPYRIDINE DERIVATIVES TO PREPARE MEDICINES FOR TREATING DISEASES CAUSING DEMYELINATION

(30) Priorité: 29.04.1997 FR 9705275
(43) Date de publication de la demande: 16.02.2000
(73) Titulaire: SANOFI-SYNTHELABO, 75635 Paris Cédex 13 (FR)
(72) Inventeur: BOURRIE, Bernard, F-34980 Saint Gély du Fesc (FR); CASELLAS, Pierre, F-34000 Montpellier (FR); MAFFRAND, Jean-Pierre, F-31120 Portet (FR)
(74) Mandataire: Thouret-Lemaitre, Elisabeth
(86) Numéro de dépôt international: PCT/FR1998/000774
(87) Numéro de publication internationale: WO 1998/048802

(56) Documents cités:
- WO-A-93/11107
- WO-A-97/01536
- FR-A- 2 740 343
- FOURNIER J ET AL: "PROTECTIVE EFFECTS OF SR 57746A IN CENTRAL AND PERIPHERAL MODELS OF NEURODEGENERATIVE DISORDERS IN RODENTS AND PRIMATES" NEUROSCIENCE, vol. 55, no. 3, 1993, pages 629-641, XP002008237
- RUIGT ET AL.: "SR 57746A attenuates cytostatic drug-induced reduction of neurite outgrowth in co-cultures of dorsal root ganglia and Schwann cells" NEUROSCI. LETT., vol. 203, no. 1, 12 janvier 1996, pages 9-12, XP002051093

## Description

La présente invention concerne l'utilisation de certaines tétrahydropyridines pour la préparation de médicaments destinés au traitement des maladies provoquant la destruction de la myéline.

Ces pathologies ont la caractéristique commune d'être d'origine inflammatoire ou autoimmune et de causer la perte de myéline dans le système nerveux central. On peut distinguer principalement entre les pathologies chroniques comme la sclérose en plaques et les pathologies aiguës comme l'encéphalomyélite disséminée aiguë et la leucoencéphalite hémorragique aiguë. Parmi ces pathologies, la sclérose en plaques est la plus répandue et conduit à des dysfonctionnements moteurs sensoriels et visuels très graves.

A présent, aucune thérapie efficace n'est disponible pour ces maladies et les traitements sont limités aux traitements symptomatiques visant l'amélioration de l'hypertonie spastique, la fatigue et la douleur ou bien, comme l'origine de ces pathologies est souvent réputée être autoimmune, visant la suppression de la réponse immunologique.

WO 93/11107 décrit une classe de N-hydroxyalkyl-1,2,3,6-tétrahydropyridines en tant que protecteurs vis-à-vis des dommages causés par l'hypoxie.

EP 0 101 381 décrit des dérivés de la trifluorométhylphényltétrahydropyridine ayant une activité anorexigène et EP 0 458 696 en décrit les effets neuroprotecteurs.

WO 97/01536 décrit des 1-phénylalkyl-1,2,3,6-tétrahydropyridines ayant également activité neurotrophique et neuroprotectrice.

Il a maintenant été trouvé que certaines tétrahydropyridines exercent une action bénéfique vis-à-vis des maladies provoquant la destruction de la myéline.
Ainsi, la présente invention a pour objet l'utilisation d'un composé de formule (I): 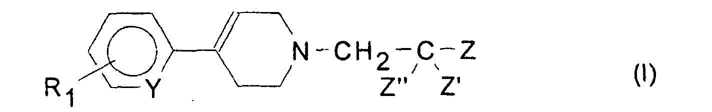 dans laquelle:
- R₁ représente un halogène ou un groupe CF₃, (C₁-C₄)alkyle ou (C₁-C₄)alcoxy;
- Y représente un atome d'azote ou un groupe CH;
- Z' et Z" représentent chacun l'hydrogène ou un groupe (C₁-C₃) alkyle, ou bien l'un représente l'hydrogène et l'autre un groupe hydroxy, ou encore les deux, ensemble, représentent un groupe oxo;
- Z représente
   ◆ un radical phényle;
   ◆ un radical phényle monosubstitué par un substituant X, X étant
      (a) un groupe (C₁-C₆)alkyle, (C₁-C₆)alcoxy, (C₃-C₇)carboxyalkyle, (C₁-C₄)alcoxycarbonyl(C₁-C₆)alkyle, (C₃-C₇)carboxyalcoxy ou (C₁-C₄)alcoxycarbonyl(C₁-C₆)alcoxy ;
      (b) un groupe choisi parmi un (C₃-C₇)cycloalkyle, (C₃-C₇)cycloalkyloxy, (C₃-C₇)cycloalkylméthyle, (C₃-C₇)cycloalkylamino et cyclohexényle, ledit groupe pouvant être substitué par un halogène, hydroxy, (C₁-C₄)alcoxy, carboxy, (C₁-C₄)alcoxycarbonyle, amino, mono- ou di-(C₁-C₄)alkylamino; ou
      (c) un groupe choisi parmi un phényle, phénoxy, phénylamino, N-(C₁-C₃)alkylphénylamino, phénylméthyle, phényléthyle, phénylcarbonyle, phénylthio, phénylsulfonyle, phénylsulfinyle et styryle, ledit groupe pouvant être mono- ou polysubstitué sur le groupe phényle par un halogène, CF₃, (C₁-C₄)alkyle, (C₁-C₄)alcoxy, cyano, amino, mono- ou di-(C₁-C₄)alkylamino, (C₁-C₄)acylamino, carboxy, (C₁-C₄)alcoxycarbonyle, aminocarbonyle, mono- ou di-(C₁-C₄)alkylaminocarbonyle, amino(C₁-C₄)alkyle, hydroxy(C₁-C₄)alkyle ou halogéno(C₁-C₄)alkyle;
   ◆ un radical phényle disubstitué par un substituant R₂, R₂ étant un halogène ou un groupe hydroxy, méthyle, éthyle, (C₃-C₆)alkyle, (C₁-C₄)alcoxy ou trifluorométhyle et par un substituant X, X étant tel que défini ci-dessus;
   ◆ un radical 1-naphtyle ou 2-naphtyle;
   ◆ un radical 1-naphtyle ou 2-naphtyle, substitué dans les positions 5, 6, 7 et/ou 8 par un ou deux groupes hydroxyle, un ou deux groupes (C₁-C₄)alcoxy, ou un groupe 6, 7-méthylènedioxy;
- ou bien Z" est l'hydrogène et Z et Z' représentent, chacun indépendamment, un groupe phényle non substitué ou mono-, di- ou trisubstitué;
ou d'un de ses sels et solvates pharmaceutiquement acceptables, pour la préparation de compositions pharmaceutiques destinées à combattre les maladies entraînant une démyélinisation.

Selon un aspect avantageux, l'invention concerne l'utilisation du composé de formule (I) dans laquelle Y est CH, R, est trifluorométhyle et Z' et Z" sont l'hydrogène ou de l'un de ses sels et solvates pharmaceutiquement acceptables.

Selon un aspect préféré, l'invention concerne l'utilisation du composé de formule (I) où Y est CH, R, est trifluorométhyle, Z' et Z" sont l'hydrogène et Z représente un groupe 2-naphtyle, 6,7-diméthoxy-2-naphtyle ou 6,7-méthylènedioxy-2-naphtyle ou de l'un de ses sels et solvates pharmaceutiquement acceptables.

Selon un autre aspect avantageux, l'invention a pour objet l'utilisation du composé de formule (I) où Y est CH, R, est trifluorométhyle, Z' et Z" sont l'hydrogène et Z représente
- soit un radical phényle monosubstitué par un substituant X, X étant:
   (a) un groupe (C₁-C₆)alkyle, (C₁-C₆)alcoxy, (C₃-C₇)carboxyalkyle, (C₁-C₄)alcoxycarbonyl(C₁-C₆)alkyle, (C₃-C₇)carboxyalcoxy ou (C₁-C₄)alcoxycarbonyl(C₁-C₆)alcoxy ;
   (b) un groupe choisi parmi un (C₃-C₇)cycloalkyle, (C₃-C₇)cycloalkyloxy, (C₃-C₇)cycloalkylméthyle, (C₃-C₇)cycloalkylamino et cyclohexényle, ledit groupe pouvant être substitué par un halogène, hydroxy, (C₁-C₄)alcoxy, carboxy, (C₁-C₄)alcoxycarbonyle, amino, mono- ou di-(C₁-C₄)alkylamino; ou
   (c) un groupe choisi parmi un phényle, phénoxy, phénylamino, N-(C₁-C₃)alkylphénylamino, phénylméthyle, phényléthyle, phénylcarbonyle, phénylthio, phénylsulfonyle, phénylsulfinyle ou styryle, ledit groupe pouvant être mono- ou polysubstitué sur le groupe phényle par un halogène, CF₃, (C₁-C₄)alkyle, (C₁-C₄)alcoxy, cyano, amino, mono- ou di-(C₁-C₄)alkylamino, (C₁-C₄)acylamino, carboxy, (C₁-C₄)alcoxycarbonyle, aminocarbonyle, mono- ou di(C₁-C₄)alkylaminocarbonyle, amino(C₁-C₄)alkyle, hydroxy(C₁-C₄)alkyle ou halogéno(C₁-C₄)alkyle,
- soit un phényle disubstitué par R₂ et X tels que définis ci-dessus,
ou de l'un de ses sels et solvates pharmaceutiquement acceptables.

Selon un autre aspect avantageux, l'invention concerne l'utilisation du composé de formule (I) dans laquelle Y est CH, R₁ est trifluorométhyle, Z' et Z" sont l'hydrogène et Z représente soit un phényle monosubstitué par un groupe X', X' étant un phényle, non substitué ou substitué par 1 à 3 halogène, 1 à 3 CF₃, 1 à 3 (C₁-C₄)alkyle, 1 à 3 (C₁-C₄)alcoxy, 1 à 3 cyano, 1 à 3 amino, 1 à 3 mono- ou di-(C₁-C₄)alkylamino, 1 à 3 (C₁-C₄)acylamino, 1 à 3 carboxy, 1 à 3 (C₁-C₄)alcoxycarbonyle, 1 à 3 aminocarbonyle, 1 à 3 mono- ou di-(C₁-C₄)alkylaminocarbonyle, 1 à 3 amino(C₁-C₄)alkyle, 1 à 3 hydroxy(C₁-C₄)alkyle ou 1 à 3 halogéno(C₁-C₄)alkyle; soit un phényle substitué par un substituant R₂, R₂ étant un halogène ou un groupe hydroxy, méthyle, éthyle, (C₃-C₆)alkyle, (C₁-C₄) alcoxy ou trifluorométhyle et par un substituant X', X' étant tel que défini ci-dessus, ou de l'un de ses sels et solvates pharmaceutiquement acceptables.

Selon un autre aspect avantageux, l'invention concerne l'utilisation du composé de formule (I) dans laquelle Y est CH, R₁ est trifluorométhyle, Z' et Z" sont l'hydrogène et Z est un groupe phényle substitué dans les positions 3 et 4 par un groupe (C₁-C₆) alkyle, ou de l'un de ses sels et solvates pharmaceutiquement acceptables.

Selon un autre aspect avantageux, l'invention concerne l'utilisation du composé de formule (I) où Y est CH, R₁ est trifluorométhyle, Z" est l'hydrogène et Z et Z', identiques, représentent chacun un groupe phényle; un groupe phényle substitué en position 2, 3 ou 4 par un atome de fluor, de chlore ou par un groupe méthyle, éthyle, *n*-propyle, *i*-propyle, *n*-butyle, *i*-butyle, *s*-butyle, *t*-butyle trifluorométhyle, cyano, méthoxy, méthylthio, méthylsulfonyle, éthoxy, éthylthio, éthylsulfonyle, (C₁-C₃)alcoxycarbonyle ou di(C₁-C₃)alkylaminocarbonyle; un groupe phényle disubstitué dans les positions 2, 4; 3, 4; 3, 5 ou 2, 6 par un atome de chlore, de fluor, ou par un groupe méthyle, éthyle, trifluorométhyle, cyano ou méthoxy; ou un groupe phényle trisubstitué dans les positions 3, 4, 5; 2, 4, 5 ou 2, 4, 6 par un atome de chlore, de fluor, ou par un groupe méthyle, éthyle, trifluorométhyle, cyano ou méthoxy ou de l'un de ses sels et solvates pharmaceutiquement acceptables.

Des composés particulièrement avantageux, selon la présente invention, sont les suivants:
- 1-(2-napht-2-yléthyl)-4-(3-trifluorométhylphényl)-1,2,3,6-tétrahydropyridine;
- 1-[2-(6,7-dimétoxynapht-2-yl)éthyl)-4-(3-trifluorométhylphényl)-1,2,3,6-tétrahydropyridine;
- 1-[2-(6,7-méthylènedioxynapht-2-yl)éthyl)-4-(3-trifluorométhylphényl)-1,2,3,6-tétrahydropyridine;
- 1-[2-(4-isobutylphényl)-propyl]-4-(3-trifluorométhylphényl)-1,2,3,6-tétrahydropyridine;
- 1-[(2S)-2-(4-isobutylphényl)propyl]-4-(3-trifiuorométhylphényl)-1,2,3,6-tétrahydropyridine;
- 1-[(2R)-2-(4-isobutylphényl)-propyl]-4-(3-trifluorométhylphényl)-1,2,3,6-tétrahydropyridine;
- 1-[2-(4-isobutylphényl)-éthyl]-4-(3-trifluorométhylphényl)-1,2,3,6-tétrahydropyridine;
- 1-[2-(4-tertbutylphényl)-éthyl]-4-(3-trifluorométhylphényl)-1,2,3,6-tétrahydropyridine;
- 1-[2-(4-isobutylphényl)-2-méthylpropyl]-4-(3-trifluorométhylphényl)-1,2,3,6-tétrahydropyridine;
- 1-[2-(4-isopropylphényl)éthyl]-4-(3-trifluorométhylphényl)-1,2,3,6-tétrahydropyridine;
- 1-[2-(3'-chloro-4-biphénylyl)éthyl]-4-(3-trifluorométhylphényl)-1,2,3,6-tétrahydropyridine;
- 1-[2-(2'-chloro-4-biphénylyl)-éthyl]-4-(3-trifluorométhyl-phényl)-1,2,3,6-tétrahydropyridine;
- 1-[2-(4'-chloro-4-biphénylyl)-éthyl]-4-(3-trifluorométhylphényl)-1,2,3,6-tétrahydropyridine;
- 1-[2-(4'-fluoro-4-biphénylyl)-éthyl]-4-(3-trifluorométhylphényl)-1,2,3,6-tétrahydropyridine;
- 1-[2-(3'-trifluorométhyl-4-biphénylyl)-éthyl]-4-(3-trifluorométhylphényl)-1,2,3,6-tétrahydropyridine;
- 1-[2-(4-cyclohexylphényl)éthyl]-4-(3-trifluorométhylphényl)-1,2,3,6-tétrahydropyridine;
- 1-[-2-(4-biphénylyl)-2-éthyl]-4-(4-fluorophényl)-1,2,3,6-tétrahydropyridine;
- 1-[2-(4-biphénylyl)-2-méthylpropyl]-4-(3-trifluorométhylphényl)-1,2,3,6-tétrahydropyridine;
- 1-[2-(4-phénoxyphényl)-2-éthyl]-4-(3-trifluorométhylphényl)-1,2,3,6-tétrahydropyridine;
- 1-[2-(4-benzylphényl)-2-éthyl]-4-(3-trifluorométhylphényl)-1,2,3,6-tétrahydropyridine;
- 1-[2-(4-n-butylphényl)éthyl]-4-(3-trifluorométhylphényl)-1,2,3,6-tétrahydropyridine;
- 1-[2-(4-biphénylyl)éthyl]-4-(3-trifluorométhylphényl)-1,2,3,6-tétrahydropyridine;
- 1-[2-(4-n-butoxyphényl)éthyl]-4-(3-trifluorométhylphényl)-1,2,3,6-tétrahydropyridine;
- 1-[2-(3,4-diéthylphényl)éthyl]-4-(3-trifluorométhylphényl)-1,2,3,6-tétrahydropyridine;
- 1-[2-(3-méthyl-4-pentylphényl)éthyl]-4-(3-trifluorométhylphényl)-1,2,3,6-tétrahydropyridine;
- 1-[2-(4-méthyl-3-pentylphényl)éthyl]-4-(3-trifluorométhylphényl)-1,2,3,6-tétrahydropyridine;
- 1-[2-(3,4-diéthylphényl)éthyl]-4-(6-chloropyrid-2-yl)-1,2,3,6-tétrahydropyridine;
- 1-(2,2-diphényléthyl)-4-(3-trifluorométhyphényl)-1,2,3,6-tétrahydropyridine
- 1-[2,2-(4,4'-dichlorodiphényl)éthyl]-4-(3-trifluorométhylphényl)-1,2,3,6-tétrahydropyridine;
- 1-[2,2-(3,3'-bistrifluorométhyldiphényl)éthyl]-4-(3-trifluorométhylphényl)-1,2,3,6-tétrahydropyridine;
- 1-[2,2-(4,4'-diméthoxydiphényl)éthyl]-4-(3-trifluorométhylphényl)-1,2,3,6-tétrahydropyridine;
- 1-[2-(4-fluorophényl)-2-phényléthyl]-4-(3-trifluorométhylphényl)-1,2,3,6-tétrahydropyridine;
- 1-(3,3-diphénylpropyl)-4-(3'-trifluorométhylphényl)-1,2,3,6-tétrahydropyridine;
- 1-[2,2-(4,4'-dichlorodiphényl)éthyl]-4-(6-chloropyrid-2-yl)-1,2,3,6-tétrahydropyridine;
- 1-[2-(3'-chlorobiphényl-4-yl)-2-oxoéthyl]-4-(3-trifluorométhylphényl)-1,2,3,6-tétrahydropyridine;
- 1-[2-(2'-chlorobiphényl-4-yl)-2-oxoéthyl]-4-(3-trifluorométhylphényl)-1,2,3,6-tétrahydropyridine;
- 1-[2-(4'-chlorobiphényl-4-yl)-2-oxoéthyl]-4-(3-trifluorométhylphényl)-1,2,3,6-tétrahydropyridine;
- 1-[2-(4-isobutylphényl)-2-oxoéthyl]-4-(3-trifluorométhylphényl)-1,2,3,6-tétrahydropyridine;
- 1-[2-(4-benzylphényl)-2-oxoéthyl]-4-(3-trifluorométhylphényl)-1,2,3,6-tétrahydropyridine;
- 1-[2-(4-cyclohexylphényl)-2-oxoéthyl]-4-(3-trifluorométhylphényl)-1,2,3,6-tétrahydropyridine;
- 1-[2-(4'-fluorobiphényl-4-yl)-2-oxoéthyl]-4-(3-trifluorométhylphényl)-1,2,3,6-tétrahydropyridine;
- 1-[2-(4-*n*-butylphényl)-2-oxoéthyl]-4-(3-trifluorométhylphényl)-1,2,3,6-tétrahydropyridine;
- 1-[2-(biphényl-4-yl)-2-oxoéthyl]-4-(3-trifluorométhylphényl)-1,2,3,6-tétrahydropyridine;
- 1-[2-(4-*t*-butylphényl)-2-oxoéthyl]-4-(3-trifluorométhylphényl)-1,2,3,6-tétrahydropyridine;
- 1-[2-(4'-trifluorométhylbiphényl-4-yl)-2-oxoéthyl]-4-(3-trifluorométhylphényl)-1,2,3,6-tétrahydropyridine;
- 1-[2-(2,3'-dichloro-4-biphénylyl)-éthyl]-4-(3-trifluorométhylphényl)-1,2,3,6-tétrahydropyridine;
- 1-[2-(3-chloro-4-biphénylyl)-éthyl]-4-(3-trifluorométhylphényl)-1,2,3,6-tétrahydropyridine;
- 1-[2-(3',5'-dichloro-4-biphénylyl)-éthyl]-4-(3-trifluorométhylphényl)-1,2,3,6-tétrahydropyridine;
- 1-[2-(2',4'-dichloro-4-biphénylyl)-éthyl]-4-(3-trifluorométhylphényl)-1,2,3,6-tétrahydropyridine;
- 1-[2-(2-chloro-4-biphénylyl)-éthyl]-4-(3-trifluorométhylphényl)-1,2,3,6-tétrahydropyridine;
- 1-[2-(3'-chloro-4-biphénylyl)-2-méthylpropyl]-4-(3-trifluorométhylphényl)-1,2,3,6-tétrahydropyridine;
- 1-[2-(2-fluoro-4-biphénylyl)-propyl]-4-(3-trifluorométhylphényl)-1,2,3,6-tétrahydropyridine;
- 1-[2-(4-méthoxy-3-biphénylyl)-éthyl]-4-(3-trifluorométhylphényl)-1,2,3,6-tétrahydropyridine;
- 1-[2-(4'-méthoxy-4-biphénylyl)-éthyl]-4-(3-trifluorométhylphényl)-1,2,3,6-tétrahydropyridine;
- 1-[2-(4'-hydroxy-4-biphénylyl)-éthyl]-4-(3-triftuorométhylphényl)-1,2,3,6-tétrahydropyridine;
- 1-[2-(4'-éthoxycarbonylbutoxy-4-biphénylyl)-éthyl]-4-(3-trifluorométhylphényl)-1,2,3,6-tétrahydropyridine;
- 1-[2-(3-biphénylyl)-éthyl]-4-(3-trifluorométhylphényl)-1,2,3,6-tétrahydropyridine;
- 1-[2-(3'chloro-4'-fluoro-4-biphénylyl)-éthyl]-4-(3-trifluorométhylphényl)-1,2,3,6-tétrahydropyridine;
- 1-[2-(2'trifluorométhyl-4-biphénylyl)-éthyl]-4-(3-trifluorométhylphényl)-1,2,3,6-tétrahydropyridine;
- 1-[2-(3,4-diisobutylphényl)-éthyl]-4-(3-trifluorométhylphényl)-1,2,3,6-tétrahydropyridine;
- 1-[2-(3,4-dipropylphényl)-éthyl]-4-(3-trifluorométhylphényl)-1,2,3,6-tétrahydropyridine;
- 1-[2-(4-cyclohexylphényl)-éthyl]-4-(6-chloropyrid-2-yl)-1,2,3,6-tétrahydropyridine;
- 1-[2-(4-isobutylphényl)-propyl]-4-(6-chloropyrid-2-yl)-1,2,3,6-tétrahydropyridine;
- 1-[2-(2'-trifluorométhylbiphényl-4-yl)-2-oxoéthyl]-4-(3-trifluorométhylphényl)-1,2,3,6-tétrahydropyridine;
- 1-[2-(3'-trifluorométhylbiphényl-4-yl)-2-oxoéthyl]-4-(3-trifluorométhylphényl)-1,2,3,6-tétrahydropyridine;
ainsi que leurs sels et solvates pharmaceutiquement acceptables.
La 1-(2-napht-2-yléthyl)-4-(3-trifluorométhylphényl)-1,2,3,6-tétrahydropyridine et ses sels et solvates pharmaceutiquement acceptables, notamment son chlorhydrate, sont des composés particulièrement préférés pour l'utilisation selon la présente invention.

Les composés de formule (I) où Z' et Z" sont l'hydrogène sont préparés comme décrit dans WO 97/01536.

Les composés de formule (I) où l'un d'entre Z' et Z" est l'hydrogène et l'autre est un hydroxyle ainsi que les composés où Z' et Z" ensemble représentent un groupe oxo peuvent être préparés comme décrit dans WO 93/11107.

Les composés de formule (I) où Z" est l'hydrogène et Z' et Z" représentent chacun indépendamment un groupe phényle non substitué, mono, di-, ou trisubstitué sont préparés selon le procédé suivant:
(a) on fait réagir une aryl-1,2,3,6-tétrahydropyridine de formule (II) 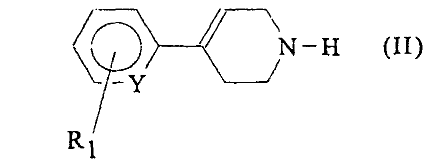 dans laquelle Y et R₁ sont tels que définis ci-dessus, avec un acide de formule (III) 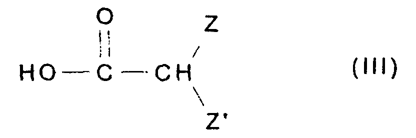 dans laquelle Z et Z' sont tels que définis ci-dessus, ou l'un de ses dérivés fonctionnels,
(b) on réduit le carbonyle intermédiaire de formule (IV), 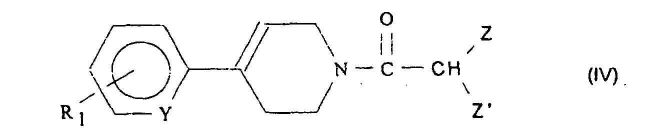
(c) on isole le composé de formule (I) ainsi obtenu et, éventuellement on le transforme en l'un de ses sels ou solvates.

La réaction de l'étape (a) peut être convenablement réalisée dans un solvant organique à une température comprise entre -10°C et la température de reflux du mélange réactionnel ; de préférence la réaction est réalisée à basse température.

Comme solvant de réaction, on utilise de préférence un solvant halogéné tel que le chlorure de méthylène, le dichloroéthane, le 1,1,1-trichloroéthane, le chloroforme et similaires ou un alcool, tel que le méthanol ou l'éthanol, mais d'autres solvants organiques compatibles avec les réactifs employés, par exemple le dioxane, le tétrahydrofurane ou un hydrocarbure tel que l'hexane, peuvent être également employés.

La réaction peut être convenablement réalisée en présence d'un accepteur de protons, par exemple d'un carbonate alcalin ou d'une amine tertiaire. Comme dérivé fonctionnel approprié de l'acide de formule (III), on peut utiliser l'acide libre, éventuellement activé (par exemple avec le BOP), l'anhydride, un anhydride mixte, un ester activé ou un halogénure d'acide, de préférence le chlorure ou bromure. Parmi les esters activés, l'ester de *p*-nitrophényle est particulièrement préféré, mais les esters de méthoxyphényle, de trityle, de benzhydryle et similaires sont également convenables.

La réduction de l'étape (b) peut être convenablement réalisée par des agents de réduction appropriés tels que les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend un solvant qui n'interfère pas avec la réaction. De tels solvants sont par exemple les éthers, tel que l'éther diéthylique, le tétrahydrofuranne, le dioxane ou le 1,2-diméthoxyéthane.

Le composé de formule (I) obtenu est isolé selon les techniques usuelles et éventuellement transformé en l'un de ses sels d'addition d'acides ou, lorsqu'un groupe acide est présent, le caractère amphotère du composé permet la séparation des sels soit avec des acides soit avec des bases.

Les amines de départ de formule (II) où Y est CH sont des composés connus ou bien elles peuvent être préparées selon des procédés analogues à ceux utilisés pour préparer les composés connus.

Les amines de départ de formule (II) où Y est N peuvent être préparées par réaction de la 2-halogénopyridine appropriée de formule (p) 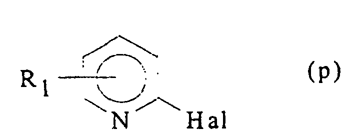 dans laquelle R₁ est tel que défini ci-dessus et Hal est un atome d'halogène,
avec une 1,2,3,6-tétrahydropyridine de formule (q) 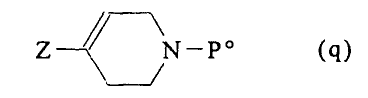 dans laquelle P° représente un groupe protecteur tel que par exemple le groupe benzyle et Z représente un substituant qui permet la substitution nucléophile de l'halogène de la pyridine. De tels substituants sont par exemple les trialkylstannanes, comme le tributylstannane ou les composés de Grignard.

On déprotège ensuite la 1,2,3,6-tétrahydropyridine par clivage du groupe protecteur dans des conditions convenables.

Les acides de formule (III) peuvent être préparés, selon la réaction de Wittig, par a) réaction entre une benzophénone appropriée de formule (r) 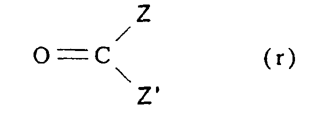 dans laquelle Z et Z' sont tels que définis ci-dessus, par réaction avec l'iodure de triméthylsulfoxonium/BF₃-Et₂O et oxydation de l'aldéhyde intermédiaire de formule (w) 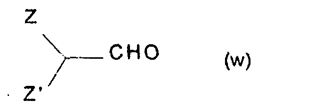 selon la méthode décrite dans J. Am. Chem. Soc., 1990, 112(18):6690-6695 pour obtenir l'acide correspondant.
Selon un autre mode opératoire, les composés de formule (I) où Z" est l'hydrogène peuvent également être préparés par réaction entre une aryl-1,2,3,6-tétrahydropyridine de formule (II) 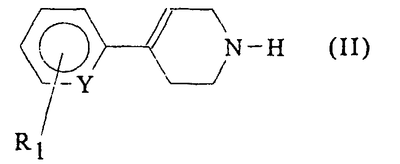 dans laquelle R₁ et Y sont tels que définis ci-dessus, et un aldéhyde de formule (w) ci-dessus en présence d'un agent de réduction tel que le cyanoborohydrure de sodium, selon les techniques connues.

Les composés de formule (I), dans laquelle R₁ est m-trifluorométhyle, Y est CH, Z' et Z" sont l'hydrogène et Z est un groupe naphtyle substitué par un ou deux groupes alcoxy ou par un groupe méthylènedioxy sont préparés comme décrit dans EP 0 458 697.

La 1-(2-napht-2-yléthyl)-4-(3-trifluorométhylphényl)-1,2,3,6-tétrahydropyridine et ses sels et solvates pharmaceutiquement acceptables, notamment le chlorhydrate, peuvent être préparés selon EP 0 101 381.

Une méthode avantageuse prévoit la réaction entre le 2-(2-bromoéthyl)naphtalène et la 4-(3-trifluorométhylphényl)-1,2,3,6-tétrahydropyridine et l'isolement de préférence du chlorhydrate de 1-(2-napht-2-yléthyl)-4-(3-trifluorométhylphényl)-1,2,3,6-tétrahydropyridine qui est par la suite cristallisé dans un mélange éthanol/eau par chauffage et refroidissement à 5°C avec une rampe de refroidissement de 10°C/heure et une vitesse d'agitation de 400 tr/minute, de façon à obtenir un melange de deux formes cristallines dans un rapport d'environ 66/34.

Le chlorhydrate de 1-(2-napht-2-yléthyl)-4-(3-trifluorométhylphényl)-1,2,3,6-tétrahydropyridine est utilisé de préférence sous forme microparticulaire, par exemple sous une forme essentiellement amorphe obtenue par atomisation ou sous une forme microsristalline obtenue par micronisation.
L'activité des composés de formule (I) a été recherchée dans le modèle d'encéphalite allergique expérimentale (EAE) induite chez le rat Lewis par l'administration intraplantaire de protéine basique de myéline (MBP) (fragment 68-84) dans un adjuvant complet de Freund (FCA) enrichi en *mycobacterium tuberculosis* selon le protocole publié par Martin et Near (Journal of Neuroimmunology, 1995, 241-245).
L'EAE est une maladie auto-immune et inflammatoire du système nerveux central présentant des lésions démyélinisantes rappelant la sclérose en plaques humaine.
Dans ce modèle expérimental, des composés représentatifs selon l'invention, administrés par voie orale depuis le jour zéro d'induction de la maladie, atténuent de façon très significative la maladie, mesurée à la fois sur les variations de poids des animaux (les animaux malades présentent une perte importante de poids) et sur la sévérité de la pathologie (les animaux malades présentent une paralysie des pattes postérieures). La perte de poids des animaux traités, notamment par le chlorhydrate de 1-(2-napht-2-yléthyl)-4-(3-trifluorométhylphényl)-1,2,3,6-tétrahydropyridine est significativement plus faible que celle des animaux traités par le seul véhicule. De même, la sévérité de la maladie est statistiquement plus faible dans les groupes d'animaux traités par le chlorhydrate de 1-(2-napht-2-yléthyl)-4-(3-trifluorométhylphényl)-1,2,3,6-tétrahydropyridine.

Un autre événement majeur reconnu dans la sclérose en plaques est la perte d'intégrité de la barrière hématoencéphalique sous attaque du système immunitaire. Cet effet pathologique est également mis en évidence dans le modèle d'EAE.

Il a été vérifié que la dégradation de la barrière hématoencéphalique est sensiblement réduite voire inexistante (la barrière ne présentant pas d'anomalie de perméabilité) chez les animaux traités par des composés représentatifs de l'invention par rapport aux animaux témoins.

Les résultats de ces études montrent que les composés de formule (I), notamment la 1-(2-napht-2-yléthyl)-4-(3-trifluorométhylphényl)-1,2,3,6-tétrahydropyridine et ses sels et solvates pharmaceutiquement acceptables, interviennent favorablement dans cette pathologie de dysfonctionnement neurologique et peuvent ainsi trouver une application clinique dans le traitement de maladies causant des lésions démyélinisantes, telle que la sclérose en plaques.

Les composés de formule (I) et leurs sels et solvates pharmaceutiquement acceptables sont de préférence administrés par voie orale.

Dans les compositions pharmaceutiques de la présente invention pour l'administration orale, le principe actif peut être administré sous formes unitaires d'administration, en mélange avec des supports pharmaceutiques classiques, aux animaux et aux êtres humains pour le traitement des affections susmentionnées. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend comme toujours du degré d'avancement de la maladie ainsi que de l'âge et du poids du patient. Néanmoins, les doses unitaires comprennent généralement de 0,1 à 100 mg, mieux de 0,25 à 50 mg, de préférence de 0,5 à 20 mg de principe actif.

Le composé préférentiel pour l'utilisation selon la présente invention, le chlorhydrate de 1-(2-napht-2-yléthyl)-4-(3-trifluorométhylphényl)-1,2,3,6-tétrahydropyridine sous forme microparticulaire est utilisé à des doses unitaires de 0,5 à 10 mg, avantageusement de 1 à 5, de préférence de 1 à 3 mg, par exemple 1 - 1,5 - 2 - 2,5 - 3 mg de principe actif. Ces doses unitaires sont administrées normalement une ou plusieurs fois par jour, de préférence une à trois fois par jour, la dose globale chez l'homme étant variable entre 0,5 et 50 mg par jour, par exemple de 1 à 20 mg par jour, avantageusement de 2 à 10 mg par jour.

Selon un autre de ses aspects, la présente invention concerne une association synergique comprenant un composé de formule (I) ou l'un de ses sels ou solvates pharmaceutiquement acceptables, et au moins un composé choisi parmi les agents immunosuppresseurs, tel que l'interféron β-1b; l'hormone adrénocorticotrope; les glucocorticoïdes tels que la prednisone ou la méthylprednisolone; les inhibiteurs de l'interleukine-1.

Plus particulièrement, l'invention concerne une association comprenant un composé de formule (I), ou l'un de ses sels ou solvates pharmaceutiquement acceptables et au moins un composé choisi parmi le roquimex (1,2-dihydro-4-hydroxy-N,1-diméthyl-2oxo-3-quinolinecarboxanilide), le myloran (produit de chez Autoimmune contenant myéline bovine), l'antegren (anticorps humain monoclonal de chez Elan/Athena Neurosciences), l'interféron β-1a recombinant.

D'autres associations possibles sont celles constituées par un composé de formule (I) ou l'un de ses sels ou solvates pharmaceutiquement acceptables et un bloqueur des canaux potassiques, tel que par exemple la fampridine (4-amino pyridine).

Selon un aspect ultérieur la présente invention concerne une méthode de traitement des maladies entraînant une démyélinisation qui comprend l'administration à un sujet en ayant besoin d'une quantité efficace d'un composé de formule (I) ou d'un de ses sels ou solvates pharmaceutiquement acceptables.

Selon un autre aspect, la présente invention a pour objet une méthode de traitement des maladies entraînant une démyélinisation qui comprend l'administration à un sujet en ayant besoin d'une quantité efficace d'une association comprenant un composé de formule (I) ou un de ses sels ou solvates pharmaceutiquement acceptables et au moins un composé choisi parmi les agents immunosuppresseurs, tel que l'interféron β-1b; l'hormone adrénocorticotrope; les glucocorticoïdes tels que la prednisone ou la méthylprednisolone; les inhibiteurs de l'interleukine-1.
Les exemples qui suivent illustrent mieux l'invention sans toutefois la limiter.

### EXEMPLE 1

### 1-(2,2-diphényléthyl)-4-(3-trifluorométhylphényl)-1,2,3,6-tétrahydropyridine et son chlorhydrate

### 1a/ 1-(α,α-diphénylacétyl)-4-(3-trifluorométhylphényl)-1,2,3,6-tétrahydropyridine

A un mélange de 8 g (0,035 mole) de 4-(3-trifluorométhylphényl)-1,2,3,6-tétrahydropyridine, 50 ml de chlorure de méthylène et 4,96 ml de triéthylamine on ajoute goutte à goutte à la température de 0/+5°C, 8 g de chlorure de α,α-diphénylacétyle dans 50 ml de chlorure de méthylène. On agite pendant une heure à la température ambiante, on évapore le solvant sous pression réduite, on reprend le résidu dans de l'éther éthylique, on lave avec une solution aqueuse d'acide chlorhydrique 0,2 M, à l'eau, avec une solution aqueuse de carbonate de sodium et encore à l'eau. On sèche sur du sulfate de sodium, on évapore le solvant sous pression réduite. On obtient 5 g du composé du titre.

### 1b/ 1-(2,2-diphényléthyl)-4-(3-trifluorométhylphényl)-1,2,3,6-tetrahydropyridine et son chlorhydrate

A un mélange de 0,7 g d'hydrure d'aluminium et de lithium dans 10 ml d'éther éthylique, on ajoute goutte à goutte à 25 °C une solution de 5 g (0,012 mole) du produit de l'étape précédente dans 50 ml d'éther éthylique. On agite à la température ambiante pendant une heure, on ajoute goutte à goutte 5 ml d'eau. On sépare les deux phases, on sèche la phase organique sur du sulfate de sodium et on évapore le solvant sous pression réduite. On obtient ainsi la 1-(2,2-diphényléthyl)-4-(3-trifluorométhylphényl)-1,2,3,6-tétrahydropyridine. On prépare le chlorhydrate à l'aide d'une solution d'éther éthylique saturée en acide chlorhydrique. On cristallise dans 150 ml d'acétate d'éthyle. P.f. (chlorhydrate) 207-210°C.

### EXEMPLE 2

### 1-[2,2-(4,4'-dichlorodiphényl)éthyl]-4-(3-trifluorométhylphényl)-1,2,3,6-tétrahydropyridine et son oxalate

### 2a/ α,α-(4,4'-dichlorodiphényl)acétaldéhyde

A un mélange de 5,5 g (0,025 mole) d'iodure de triméthylsulfoxonium dans 10 ml de tétrahydrofuranne anhydre on ajoute par portions 0,75 g (0,025 mole) d'hydrure de sodium à 80% dans l'huile. On chauffe à 55°C pendant 6 heures et on y ajoute 6 g (0,025 mole) de 4,4'-dichlorobenzophénone dans 10 ml de tétrahydrofuranne anhydre. On laisse agiter le mélange à 55°C pendant une nuit, on verse dans l'eau, on extrait à l'éther éthylique, on sèche la phase organique sur du sulfate de sodium, on évapore le solvant sous pression réduite. On dissout le résidu dans 32 ml de toluène et on y ajoute 3 ml de BF₃-Et₂O. On agite pendant 2 minutes puis on laisse reposer pendant 3 minutes. On lave deux fois avec une solution aqueuse de bicarbonate de sodium, on sèche la phase organique sur du sulfate de sodium, on évapore le solvant sous pression réduite. On obtient une huile qu'on purifie par chromatographie sur colonne de gel de silice en éluant par un mélange hexane/acétate d'éthyle = 9/1. On obtient le composé du titre.

### 2b/ 1-[2,2-(4,4'-dichlorodiphényl)éthyl]-4-(3-trifluorométhylphényl)-1,2,3,6-tétrahydropyridine et son oxalate

A la température de 0/+5°C on mélange 1,3 g (0,0045 mole) du produit de l'étape précédente, 1,2 g (0,0053 mole) de 4-(3-trifluorométhylphényl)-1,2,3,6-tétrahydropyridine, 21 ml de méthanol, 0,8 ml d'acide acétique glacial et 0,5 g d'acétate de sodium anhydre. A la même température on ajoute au mélange 0,76 g (0,0121 mole) de cyanoborohydrure de sodium, on agite pendant 1,5 heures à basse température, puis à la température ambiante pendant une nuit. On ajoute goutte à goutte 5 ml d'acide chlorhydrique concentré, on laisse agiter pendant 10 minutes, on évapore le méthanol et on reprend le résidu dans un mélange acétate d'éthyle/solution aqueuse de NH₄OH diluée. On sépare les deux phases, on sèche la phase organique sur du sulfate de sodium et on évapore le solvant sous pression réduite. On obtient une huile qu'on purifie par chromatographie sur colonne de gel de silice en éluant par un mélange hexane/acétate d'éthyle = 9/1. On obtient le composé du titre sous forme de base. On prépare l'oxalate à l'aide d'acide oxalique dans de l'isopropanol. P.f. (oxalate) 187-189°C.

### EXEMPLE 3

### 1-[2,2-(3,3'-bistrifluorométhyldiphényl)éthyl]-4-(3-trifluorométhylphényl)-1,2,3,6-tétrahydropyridine et son oxalate

### 3a/ α,α-(3,3'-bistrifluorométhyldiphényl)acétaldéhyde

En opérant comme décrit dans l'exemple 2a/, mais en utilisant la 3,3'-bistrifluorométhylbenzophénone on obtient le composé du titre.

### 3b/ 1-[2,2-(3,3'-bistrifluorométhyldiphényl)éthyl]-4-(3-trifluorométhylphényl)-1,2,3,6-tétrahydropyridine et son oxalate

En opérant comme décrit dans l'exemple 2b/, mais en utilisant le produit de l'étape précédente au lieu de l' α,α-(4,4'-dichlorodiphényl)acétaldéhyde on obtient les composés du titre. P.f. (oxalate) 194-196°C.

### EXEMPLE 4

### 1-[2,2-(4,4'-diméthoxydiphényl)éthyl]-4-(3-trifluorométhylphényl)-1,2,3,6-tétrahydropyridine et son chlorhydrate

### 4a/ α,α-(4,4'-diméthoxydiphényl)acétaldéhyde

En opérant comme décrit dans l'exemple 2a/, mais en utilisant la 4,4'-diméthoxybenzophénone on obtient le composé du titre.

### 4b/ 1-[2,2-(4,4'-diméthoxydiphényl)éthyl]-4-(3-trifluorométhylphényl)-1,2,3,6-tétrahydropyridine et son chlorhydrate

En opérant comme décrit dans l'exemple 2b/, mais en utilisant le produit de l'étape précédente au lieu de l' α,α-(4,4'-dichlorodiphényl)acétaldéhyde on obtient les composés du titre. P.f. (chlorhydrate) 214-216°C.

### EXEMPLE 5

### 1-[2-(4-fluorophényl)-2-phényléthyl]-4-(3-trifluorométhylphényl)-1,2,3,6-tétrahydropyridine et son chlorhydrate

### 5a/ α-4-fluorophényl-α-phénylacétaldéhyde

En opérant comme décrit dans l'exemple 2a/, mais en utilisant la 4-fluorobenzophénone on obtient le composé du titre.

### 5b/ 1-[2,2-(4-fluorophényl)-2 phényléthyl]-4-(3-trifluorométhylphényl)-1,2,3,6-tétrahydropyridine et son chlorhydrate

En opérant comme décrit dans l'exemple 2b/, mais en utilisant le produit de l'étape précédente au lieu de l' α,α-(4,4'-dichlorodiphényl)acétaldéhyde on obtient les composés du titre. P.f. (chlorhydrate) 206-208°C.

### EXEMPLE 6

### 1-(3,3-diphénylpropyl)-4-(3-trifluorométhylphényl)-1,2,3,6-tétrahydropyridine et son chlorhydrate

En opérant comme décrit dans l'exemple 1b/ mais en utilisant l'acide 3,3-diphénylpropionique commercial (Aldrich, référence D21,165-6) au lieu de l'acide 2,2-diphénylacétique, on obtient les composés du titre. P.f. (chlorhydrate) 176-178°C.

### EXEMPLE 7

### 1-[2,2-(4,4'-dichlorodiphényl)éthyl]-4-(6-chloropyrid-2-yl)-1,2,3,6-tétrahydropyridine et son chlorhydrate

En opérant comme décrit dans l'exemple 2b/ mais en utilisant la 4-(6-chloropyrid-2-yl)-1,2,3,6-tétrahydropyridine au lieu de la 4-(3-trifluorométhylphényl)-1,2,3,6-tétrahydropyridine, on obtient le composé du titre. P.f. (chlorhydrate) 230-32°C.

### EXEMPLE 8

### Chlorhydrate de 1-[2-(3,4-diéthylphényl)éthyl]-4-(3-trifluorométhylphényl)-1,2,3,6-tétrahydropyridine.

### 8a/ 1-bromo-2-(3,4-diéthylphényl)éthane.

On refroidit à 0-5°C un mélange de 4,4 g (0,033 mole) de 3,4-diéthylbenzène, 50 ml de chlorure de méthylène, 8,8 g (0,044 mole) de bromure de bromoacétyle et on y ajoute 5,0 g (0,037 mole) de trichlorure d'aluminium. On agite à 0-5°C pendant une heure puis on laisse une nuit à la température ambiante. On verse dans un mélange eau/glace, on extrait au chlorure de méthylène, on sèche la phase organique sur du sulfate de sodium et on évapore le solvant sous pression réduite. On mélange 2,9 g (0,011 mole) de l'huile ainsi obtenue avec 6 ml (0,079 mole) d'acide trifluoroacétique et 6,7 ml (0,057 mole) de triéthylsilane et on chauffe à 80°C pendant 4 heures. On ajoute ensuite une solution aqueuse saturée de bicarbonate de sodium jusqu'à pH basique, on extrait à l'éther éthylique, on sèche la phase organique sur du sulfate de sodium et on évapore le solvant sous pression réduite. On purifie l'huile brute ainsi obtenue par chromatographie sur colonne de gel de silice en éluant avec du cyclohexane. On obtient le composé du titre.

### 8b/ Chlorhydrate de 1-[2-(3,4-diéthylphényl)éthyl]-4-(3-trifluorométhylphényl)-1,2,3,6-tétrahydropyridine.

On chauffe au reflux pendant 5 heures un mélange de 2,6 g (0,001 mole) de 4-(3-trifluorométhylphényl)-1,2,3,6-tétrahydropyridine, 60 ml de butanol, 4,1 g (0,025 mole) de carbonate de potassium anhydre râpé et 2,6 g (0,00113 mole) du produit de l'étape précédente. On évapore le solvant sous pression réduite, on reprend à l'acétate d'éthyle, on lave à l'eau, on sèche sur du sulfate de sodium et on évapore le solvant sous pression réduite. On prépare le chlorhydrate de l'huile ainsi obtenue par traitement avec une solution d'isopropanol saturée en acide chlorhydrique. On obtient 1,6 g du composé du titre. P.f. 220-222°C.

### EXEMPLE 9

### 1-[2-(3-méthyl-4-pentylphényl)éthyl]-4-(3-trifluorométhylphényl)-1,2,3,6-tétrahydropyridine et 1-[2-(4-méthyl-3-pentylphényl)éthyl]-4-(3-trifluorométhylphényl)-1,2,3,6-tétrahydropyridine et leurs oxalates.

### 9a/ 1-méthyl-2-pentylbenzène.

A une solution de 50 ml (0,1 mole) d'une solution de chlorure de n-butylmagnésium 2M dans le THF sous atmosphère d'azote, on ajoute goutte à goutte 4,7 g (0,035 mole) d'aldéhyde phtalique. Le mélange se chauffe spontanément à 40-45°C. On agite à la température ambiante pendant une heure, on verse dans une solution saturée de chlorure d'ammonium. On extrait à l'éther éthylique, on lave à l'eau, on sèche sur du sulfate de sodium et on évapore le solvant sous pression réduite. On purifie l'huile ainsi obtenue par chromatographie sur colonne de gel de silice en éluant par un mélange cyclohexane/acétate d'éthyle = 7/3. On isole le produit ayant le Rf le plus élevé. On obtient 2,0 g d'huile. On dissout le brut de réaction dans 25 ml d'éthanol et on y ajoute 1 ml d'acide sulfurique concentré et 0,15 g de Pd/C à 10%. On hydrogène à la température ambiante pendant 7 heures. On filtre le catalyseur, on évapore le solvant sous pression réduite et on reprend le résidu à l'acétate d'éthyle. On lave avec une solution aqueuse de bicarbonate de sodium, on sèche et on évapore le solvant sous pression réduite. On obtient 1,35 g du produit du titre.

### 9b/ 1-bromo-2-(3-méthyl-4 pentylphényl)éthane et 1-bromo-2-(4-méthyl-3-pentylphényl)éthane.

On refroidit à 0-5°C un mélange de 1,17 g (0,0054 mole) du produit de l'étape précédente, 0,62 ml (0,0072 mole) de bromure de bromoacétyle et on y ajoute 0,81 g (0,006 mole) de trichlorure d'aluminium. On agite à 0-5°C pendant une heure et ensuite 4 heures à la température ambiante. On verse dans de la glace, on sépare les deux phases, on lave la phase organique à l'eau, on la sèche et on évapore le solvant sous pression réduite. On dissout le résidu dans 2,9 ml d'acide trifluoroacétique et on y ajoute 3,1 ml (0,0267 mole) de triéthylsilane et on chauffe le mélange à 80°C pendant 5 heures. On verse dans une solution aqueuse de bicarbonate de sodium et on extrait à l'éther éthylique. On lave à l'eau, on sèche sur du sulfate de sodium. On obtient un mélange des composés du titre.

### 9c/ 1-[2-(3-méthyl-4-pentylphényl)éthyl]-4-(3-trifluorométhylphényl)-1,2,3,6-tétrahydropyridine et 1-[2-(4-méthyl-3-pentylphényl)éthyl]-4-(3-trifluorométhylphényl)-1,2,3,6-tétrahydropyridine et leurs oxalates.

On chauffe au reflux pendant 6 heures un mélange de 0,7 g (0,0031 mole) de 4-(3-trifluorométhylphényl)-1,2,3,6-tétrahydropyridine, 16 ml de butanol, 0,9 g (0,0065 mole) de carbonate de potassium anhydre râpé et le produit obtenu à l'étape précédente (0,0054 mole théorique). On évapore le solvant sous pression réduite, on reprend à l'acétate d'éthyle, on lave à l'eau, on sèche sur du sulfate de sodium et on évapore le solvant sous pression réduite. On purifie l'huile ainsi obtenue par chromatographie sur colonne de gel de silice en éluant par un mélange cyclohexane/acétate d'éthyle = 7/3. On isole deux produits ayant un Rf similaire. Le produit ayant le Rf le plus élevé correspond à la 1-[2-(3-méthyl-4-pentylphényl)éthyl]-4-(3-trifluorométhylphényl)-1,2,3,6-tétrahydropyridine. On prépare l'oxalate dans de l'acétone. On obtient 0,12 g de produit. P.f. 140-143°C. Le produit ayant le Rf le plus faible correspond à l'isomère 1-[2-(4-méthyl-3-pentylphényl)éthyl]-4-(3-trifluorométhylphényl)-1,2,3,6-tétrahydropyridine. On prépare l'oxalate dans de l'acétone. On cristallise le produit dans de l'acétone. On obtient 0,08 g de produit. P.f. 167-169°C.

### EXEMPLE 10

### Chlorhydrate de 1-[2-(3,4-diéthylphényl)éthyl]-4-(6-chloropyrid-2-yl)-1,2,3,6-tétrahydropyridine.

### 10a/ (1-benzyl-1,2,3,6-tétrahydropyrid-4yl)tributylstannane.

On agite à la température ambiante pendant 3 heures un mélange de 15,85 g (0,0837 mole) de 1-benzyl-4-pipéridone dans 140 ml de diméthoxyéthane anhydre et 25 g (0,0837 mole) de trisilidrazine dans 140 ml de diméthoxyéthane anhydre. On évapore le solvant sous pression réduite. On reprend le résidu dans 420 ml d'hexane anhydre et on y ajoute 420 ml de tétraméthyléthylènediamine anhydre. On refroidit le mélange à -78°C et on y ajoute goutte à goutte 156 ml de n-butyl lithium (0,25 mole) (solution 1,6 M dans l'hexane). Après environ 30 minutes on laisse revenir la température jusqu'à 0°C et on agite pendant 15 minutes. On ajoute ensuite au mélange réactionnel 45 ml (0,167 mole) de chlorure de tributylstannane. Après 1 heure on ajoute, avec extrême précaution, un mélange eau/glace. On extrait à l'éther éthylique, on lave la phase organique à l'eau, on sèche sur du sulfate de sodium et on évapore le solvant sous pression réduite. On obtient 70 g de produit brut qu'on purifie par chromatographie sur colonne de gel de silice en éluant avec un mélange cyclohexane/acétate d'éthyle = 95/5. On obtient le composé du titre sous forme d'huile.
¹H-RMN (CDCl₃) - δ (ppm) : 0,84 (9H; m: CH₃); 1,19-1,58 (18H; m: CH₂ - chaîne); 2,31 (2H; m); 2,53 (2H; m); 3,02 (2H; m); 3,56 (2H; s: méthylène benzylique); 5,76 (1H; m*); 7,18-7,41 (5H; m: arom.)
* bandes satellites ³J_{cis}(¹H-¹¹⁷Sn) et ³J_{cis}(¹H-¹¹⁹Sn).

### 10b/ 1-benzyl-4-(6-chloropyrid-2-yl)-1,2,3,6-tétrahydropyridine.

On dissout 18,5 g (0,04 mole) du composé de l'étape précédente dans 200 ml de diméthylformamide anhydre sous atmosphère d'azote. On ajoute à la solution 11,8 g (0,08 mole) de 2,6-dichloropyridine, 0,64 g de Pd(II) (Ph₃P)₂Cl₂, 4,38 g (0,04 mole) de chlorure de tétraméthylammonium et 2,76 g (0,02 mole) de carbonate de potassium. On chauffe à 110°C pendant 6 heures puis on verse le mélange dans 100 ml d'une solution d'acide sulfurique à 5%. On extrait à l'éther éthylique, on ajoute de l'hydroxyde d'ammonium à la phase aqueuse juqu'à pH basique et on extrait à l'acétate d'éthyle. On sèche les phases organiques réunies sur du sulfate de sodium et on évapore le solvant sous pression réduite. On purifie le résidu par chromatographie sur colonne de gel de silice en éluant avec un mélange cyclohexane/acétate d'éthyle = 1/1. On obtient le composé du titre. P.f. 100-102°C.

### 10c/ Chlorhydrate de 4-(6-chloropyrid-2-yl)-1,2,3,6-tétrahydropyridine.

On refroidit à 0-5°C une solution de 7,0 g (0,024 mole) du composé de l'étape précédente dans 110 ml de dichloroéthane et on y ajoute 5,8 ml (0,054 mole) de chloroformiate de chloroéthyle. On agite pendant 5 minutes puis on chauffe au reflux pendant 1,5 heures. On évapore le solvant sous pression réduite, on reprend le résidu dans 100 ml de méthanol et on chauffe au reflux pendant 1 heure. On évapore le solvant, on reprend le résidu dans de l'isopropanol et on filtre le solide. On obtient le composé du titre qu'on cristallise dans de l'éthanol à 90%. P.f. 305-307°C.

### 10d/ Chlorhydrate de 1-[2-(3,4-diéthylphényl)éthyl]-4-(6-chloropyrid-2 yl)-1,2,3,6-tétrahydropyridine.

En opérant comme décrit dans l'exemple 8b/ mais en utilisant le produit de l'étape précédente au lieu de la 4-(3-trifluorométhylphényl)-1,2,3,6-tétrahydropyridine, on obtient le composé du titre. P.f. 234-236°C.

### EXEMPLES 11-20

En opérant comme décrit dans l'exemple 9 mais en utilisant l'halogénure de magnésium approprié, on obtient les composés suivants:
**1-[2-(3-éthyl-4-méthylphényl)éthyl]-4-(3-trifluorométhylphényl)-1,2,3,6-tétrahydropyridine - Ex. 11**
**1-[2-(4-éthyl-3-méthylphényl)éthyl]-4-(3-trifluorométhylphényl)-1,2,3,6-tétrahydropyridine - Ex. 12**
**1-[2-(3-éthyl-4-propylphényl)éthyl]-4-(3-trifluorométhylphényl)-1,2,3,6-tétrahydropyridine - Ex. 13**
**1-[2-(4-éthyl-3-propylphényl)éthyl]-4-(3-trifluorométhylphényl)-1,2,3,6-tétrahydropyridine - Ex. 14**
**1-[2-(3-butyl-4-méthylphényl)éthyl]-4-(3-trifluorométhylphényl)-1,2,3,6-tétrahydropyridine - Ex. 15**
**1-[2-(4-butyl-3-méthylphényl)éthyl]-4-(3-trifluorométhylphényl)-1,2,3,6-tétrahydropyridine - Ex. 16**
**1-[2-(3-isobutyl-4-méthytphényl)éthyl]-4-(3-trifluorométhylphényl)-1,2,3,6-tétrahydropyridine - Ex. 17**
**1-[2-(4-*iso*butyl-3-méthylphényl)éthyl]-4-(3-trifluorométhylphényl)-1,2,3,6-tétrahydropyridine - Ex. 18**
**1-[2-(3-isobutyl-4-éthylphényl)éthyl]-4-(3-trifluorométhylphényl)-1,2,3,6-tétrahydropyridine - Ex.19**
**1-[2-(4-*iso*butyl-3-éthylphényl)éthyl]-4-(3-trifluorométhylphényl)-1,2,3,6-tétrahydropyridine - Ex. 20**

### EXEMPLE 21

### 1-[2-(6-méthyl-3-biphénylyl)éthyl]-4-(3-tritluorométhylphényl)-1,2,3,6-tétrahydropyridine

En opérant comme décrit dans l'exemple 9 mais en utilisant le phényl-lithium au lieu du chlorure de n-butylmagnésium, on obtient le composé du titre.

### EXEMPLE 22

### Chlorhydrate de 1-[2-(3'-chlorobiphényl-4-yl)-2-oxoéthyl]-4-(3-trifluorométhylphényl)-1,2,3,6-tétrahydropyridine.

### 22a/ 1-bromo-2-(3'-chlorobiphényl-4-yl)éthanone.

On refroidit à 0-5°C un mélange de 5 g (0,026 mole) de 3-chlorobiphényle, 50 ml de chlorure de méthylène, 6,95 g (0,034 mole) de bromure de bromoacétyle et on y ajoute 4 g (0,030 mole) de trichlorure d'aluminium. On agite pendant 1 heure à 5°C puis 4 heures à la température ambiante. On verse dans un mélange eau/glace, on extrait au chlorure de méthylène, on lave la phase organique avec une solution 1N de HCl, on sèche sur du sulfate de sodium et on évapore sous pression réduite. On obtient 4,5 g du produit du titre. P.f. 63-65°C.

### 22b/ Chlorhydrate de 1-[2-(3'-chlorobiphényl-4-yl)-2-oxoéthyl]-4-(3-trifluorométhylphényl)-1,2,3,6-tétrahydropyridine

On chauffe au reflux pendant 1 heure un mélange de 0,4 g (0,013 mole) du produit de l'étape précédente, 2,95 g (0,013 mole) de 4-(3-trifluorométhylphényl)-1,2,3,6-tétrahydropyridine, 80 ml d'éthanol et 2,32 g (0,0167 mole) de carbonate de potassium anhydre râpé. On élimine les sels par filtration, et on acidifie la solution par addition d'une solution d'éthanol saturée en acide chlorhydrique. On concentre sous pression réduite jusqu'à environ 40 ml et on laisse une nuit à 5°C. On filtre le précipité, on le lave à l'eau et ensuite avec de l'isopropanol. On obtient 4,9 g du composé du titre. P.f. 217-220°C.

### EXEMPLE 23

### Chlorhydrate de 1-[2-(2'-chlorobiphényl-4-yl)-2-oxoéthyl]-4-(3-trifluorométhylphényl)-1,2,3,6-tétrahydropyridine.

En opérant comme décrit dans l'exemple 22 mais en utilisant le 2-chlorobiphényle au lieu du 3-chlorobiphényle, on obtient le composé du titre. P.f. 200-202°C (cristallisé dans l'isopropanol).

### EXEMPLE 24

### Chlorhydrate de 1-[2-(4'-chlorobiphényl-4-yl)-2-oxoéthyl]-4-(3-trifluorométhylphényl)-1,2,3,6-tétrahydropyridine.

En opérant comme décrit dans l'exemple 22 mais en utilisant le 4-chlorobiphényle au lieu du 3-chlorobiphényle, on obtient le composé du titre. P.f. 210-215°C.

### EXEMPLE 25

### Chlorhydrate de 1-[2-(4-isobutylphényl)-2-oxoéthyl]-4-(3-trifluorométhylphényl)-1,2,3,6-tétrahydropyridine.

En opérant comme décrit dans l'exemple 22 mais en utilisant le 4-isobutylbenzène au lieu du 3-chlorobiphényle, on obtient le composé du titre. P.f. 224-228°C (cristallisé dans l'isopropanol).

### EXEMPLE 26

### Chlorhydrate de 1-[2-(4-phénoxyphényl)-2-oxoéthyl]-4-(3-trifluorométhylphényl)-1,2,3,6-tétrahydropyridine.

En opérant comme décrit dans l'exemple 22 mais en utilisant le diphényléther au lieu du 3-chlorobiphényle, on obtient le composé du titre. P.f. 205-210°C

### EXEMPLE 27

### Chlorhydrate de 1-[2-(4-cyclohexylphényl)-2-oxoéthyl]-4-(3-trifluorométhylphényl)-1,2,3,6-tétrahydropyridine.

En opérant comme décrit dans l'exemple 22 mais en utilisant le cyclohexylbenzène au lieu du 3-chlorobiphényle, on obtient le composé du titre. P.f. 209-213°C (cristallisé dans l'isopropanol).

### EXEMPLE 28

### Chlorhydrate de 1-[2-(4'-fluorobiphényl-4-yl)-2-oxoéthyl]-4-(3-trifluorométhylphényl)-1,2,3,6-tétrahydropyridine.

En opérant comme décrit dans l'exemple 22 mais en utilisant le 4-fluorobiphényle au lieu du 3-chlorobiphényle, on obtient le composé du titre. P.f. 123-125°C (cristallisé dans l'isopropanol).

### EXEMPLE 29

### Chlorhydrate de 1-[2-(biphényl-4-yl)-2-oxoéthyl]-4-(3-trifluorométhylphényl)-1,2,3,6-tétrahydropyridine.

En opérant comme décrit dans l'exemple 22 mais en utilisant le biphényle au lieu du 3-chlorobiphényle, on obtient le composé du titre. P.f. 145-147°C (base); P.f. 240-243°C (chlorhydrate).

### EXEMPLE 30

### Chlorhydrate de 1-[2-(4-n-butylphényl)-2-oxoéthyl]-4-(3-trifluorométhylphényl)-1,2,3,6-tétrahydropyridine.

En opérant comme décrit dans l'exemple 22 mais en utilisant le 4-*n*-butylbenzène au lieu du 3-chlorobiphényle, on obtient le composé du titre. P.f. 218-221°C.

### EXEMPLE 31

### Chlorhydrate de 1-[2-(4-t-butylphényl)-2-oxoéthyl]-4-(3-trifluorométhylphényl)-1,2,3,6-tétrahydropyridine.

En opérant comme décrit dans l'exemple 22 mais en utilisant le 4-*t*-butylbenzène au lieu du 3-chlorobiphényle, on obtient le composé du titre. P.f. 97-99°C (base).

### EXEMPLE 32

### Chlorhydrate de 1-[2-(3,4-diéthylphényl)-2-oxoéthyl]-4-(3-trifluorométhylphényl)-1,2,3,6-tétrahydropyridine.

En opérant comme décrit dans l'exemple 22 mais en utilisant le 3,4-diéthylbenzène au lieu du 3-chlorobiphényle, on obtient le composé du titre. P.f. 232-234°C.

### EXEMPLE 33

### Chlorhydrate de 1-[2-(2'-trifluorométhylbiphényl-4-yl)-2-oxoéthyl]-4-(3-trifluorométhylphényl)-1,2,3,6-tétrahydropyridine.

### 33a/ 2-(4-bromophényl)-2,2-diméthoxyéthane

On chauffe au reflux pendant 3 heures un mélange de 2 g (0,01 mole) de 4-bromoacétophénone, 5,6 ml d'orthoformiate de triméthyle, 5,6 ml de méthanol et 0,67 g d'Amberlite ® IR 120. Après refroidissement, on filtre sur Célite ® et on évapore la solution filtrée. On obtient 2,4 g du produit du titre sous forme huileuse.

### 33b/ 2,2-diméthoxy-2-(2'-trifluorométhylbiphényl-4-yl)éthane

On agite à 70°C pendant 1 heure, un mélange de 4,9 g (14 mmole) du produit de l'étape précédente, 2,45 g (16 mmole) d'acide 2-trifluorométhylbenzèneboronique, 63 mg (0,28 mmole) d'acétate de palladium, 4,84 g (35 mmole) de carbonate de potassium et 4,5 g (14 mmole) de bromure de tétrabutylammonium dans 19 ml d'eau. On laisse refroidir et on extrait à l'acétate d'éthyle. On sèche la phase organique sur du sulfate de sodium, on filtre et on évapore le solvant sous pression réduite. On obtient le composé du titre sous forme huileuse.

### 33c/ 4-(2-trifluorophényl)acétophénone

A une solution de 4,6 g (0,0105 mole) du produit de l'étape précédente dans 4 ml de chlorure de méthylène, on ajoute à 0°C une solution de 4 ml d'acide trifluoroacétique et 4 ml d'eau. On agite à la température ambiante pendant 2 heures, on verse dans de l'eau, on extrait au chlorure de méthylène. On sèche la phase organique, on filtre et on évapore le solvant sous pression réduite. On purifie le brut par chromatographie sur colonne de gel de silice en éluant avec un mélange cyclohexane/acétate d'éthyle = 9/1. On obtient 1,97 g du produit du titre.

### 33d/ α-bromo-4-(2-trifluorométhylphényl)acétophénone

A une solution de 1,97 g (7,5 mmole) du produit de l'étape précédente dans 5,4 ml de méthanol, on ajoute goutte à goutte à la température de 0°C, 0,38 ml (7,5 mmole) de brome. On agite à la température ambiante pendant 3 heures, on évapore le solvant, on reprend le résidu dans de l'eau et on extrait à l'acétate d'éthyle. On sèche la phase organique sur du sulfate de sodium, on filtre et on évapore le solvant sous pression réduite. On obtient le produit du titre sous forme huileuse.

### 33e/ Chlorhydrate de 1-[2-(2'-trifluorométhylbiphényl-4 yl)-2-oxoéthyl]-4-(3-trifluorométhylphényl)-1,2,3,6-tétrahydropyridine

On chauffe au reflux pendant 1 heure un mélange de 0,74 g (0,0028 mole) de 4-(3-trifluorométhylphényl)-1,2,3,6-tétrahydropyridine, 14 ml d'éthanol et 1,27 g (0,0092 mole) de carbonate de potassium anhydre râpé. On y ajoute une solution de 1,2 g (0,0035 mole) de l'huile de l'étape précédente dans 3 ml d'éthanol et on laisse au reflux pendant 30 minutes. On élimine les sels par filtration, et on acidifie la solution par addition d'une solution aqueuse d'acide chlorhydrique 1 N. On évapore le solvant sous pression réduite, on extrait au chloroforme, on sèche la phase organique sur du sulfate de sodium, on filtre et on évapore le solvant sous pression réduite. On libère la base à l'aide d'une solution d'ammoniaque concentrée, on extrait à l'acétate d'éthyle, et on purifie le produit par chromatographie sur colonne de gel de silice en éluant avec un mélange cyclohexane/acétate d'éthyle = 8/2 On obtient le composé du titre. On prépare le chlorhydrate à l'aide d'une solution d'isopropanol saturée en acide chlorhydrique. P.f. 195-197°C.

### EXEMPLE 34

### Chlorhydrate de 1-[2-(3'-trifluorométhylbiphényl-4-yl)-2-oxoéthyl]-4-(3-trifluorométhylphényl)-1,2,3,6-tétrahydropyridine.

En opérant comme décrit dans l'exemple 33 mais en utilisant l'acide 3-trifluorométhylbenzèneboronique au lieu de l'acide 2-trifluorométhylbenzèneboronique dans l'étape 12b/, on obtient le composé du titre. P.f. 232-234°C.

### EXEMPLE 35

### Chlorhydrate de 1-[2-(4'-trifluorométhylbiphényl-4-yl)-2-oxoéthyl]-4-(3-trifluorométhylphényl)-1,2,3,6-tétrahydropyridine.

En opérant comme décrit dans l'exemple 33 mais en utilisant l'acide 4-trifluorométhylbenzèneboronique au lieu de l'acide 2-trifluorométhylbenzèneboronique dans l'étape 12b/, on obtient le composé du titre. P.f. 245-247°C.

### EXEMPLE 36

Un mélange de 12,5 g de 2-(2-bromoéthyl)naphtalène, 14 g de chlorhydrate de 4-(3-trifluorométhylphényl)-1,2,3,6-tétrahydropyridine, 4,34 g d'hydroxyde de sodium, 135 ml d'eau et 95 ml d'éthanol 95% est chauffé pendant 5 heures au reflux, puis on laisse le mélange réactionnel refroidir pendant une nuit à la température ambiante. On refroidit le mélange au dessous de 25°C, puis on filtre, on lave à l'eau le produit ainsi isolé et on le sèche sous vide à 50 °C. On obtient ainsi la 1-[2-(2-naphtyl)éthyl]-4-(3-trifluorométhylphényl)-1,2,3,6-tétrahydropyridine base avec un rendement de 90 % calculé sur le chlorhydrate de 4-(3-trifluorométhylphényl)-1,2,3,6-tétrahydropyridine de départ.

### EXEMPLE 37

Un mélange de 19,5 g de chlorhydrate de 1-[2-(2-naphtyl)éthyl]-4-(3-trifluorométhylphényl)-1,2,3,6-tétrahydropyridine brut, 95 ml d'éthanol absolu et 4,65 ml d'acide chlorhydrique à 37% est chauffé au reflux sous agitation jusqu'à dissolution complète, puis on laisse refroidir, toujours sous agitation. Lorsque les premiers cristaux commencent à se former (vers 63°C), l'agitation est arrêtée et le mélange réactionnel est maintenu à 0-5°C pendant une nuit. Après filtration, le produit est réempâté deux fois dans 30 ml d'éthanol absolu, puis séché pendant une nuit à 40°C sous vide.
Dans ces conditions, on a obtenu 12,8 g de Forme I du chlorhydrate de 1-[2-(2-naphtyl)éthyl]-4-(3-trifluorométhylphényl)-1,2,3,6-tétrahydropyridine.
A l'analyse calorimétrique différentielle, la Forme I obtenue dans cette préparation a présenté
· une température de transition solide-solide de 148-149°C
· une enthalpie de transition de 26,4 J/g.

### EXEMPLE 38

Dans un réacteur calorimétrique METTLER RC1 muni d'un agitateur à palette ("impeller") de 8 cm de diamètre, un mélange de 70 g de chlorhydrate de 1-[2-(2-naphtyl)éthyl]-4-(3-trifluorométhylphényl)-1,2,3,6-tétrahydropyridine brut et de 1 1 d'éthanol absolu est chauffé au reflux jusqu'à dissolution complète du produit. La solution ainsi obtenue est refroidie avec une vitesse de refroidissement de 80°C par heure et une vitesse d'agitation de 500 tr/minute jusqu'à 10°C. Le précipité ainsi obtenu est filtré et séché une nuit à 45°C sous vide.
Dans ces conditions, on a obtenu la Forme II du chlorhydrate de 1-[2-(2-naphtyl)éthyl]-4-(3-trifluorométhylphényl)-1,2,3,6-tétrahydropyridine.
A l'analyse calorimétrique différentielle, le Forme II obtenue dans cette préparation a présenté
· une température de transition solide-solide de 153-155°C
· une enthalpie de transition de 24,1 J/g.

### EXEMPLE 39

Un mélange de 2 g de chlorhydrate de 1-[2-(2-naphtyl)éthyl]-4-(3-trifluorométhylphényl)-1,2,3,6-tétrahydropyridine et de 50 ml de diméthylsulfoxyde est chauffé au reflux jusqu'à dissolution complète, on laisse refroidir le mélange pendant une nuit, puis on récupère le produit cristallin et on le sèche sous vide à 45° C pendant une nuit.
Dans ces conditions, on a obtenu la Forme III du chlorhydrate de 1-[2-(2-naphtyl)éthyl]-4-(3-trifluorométhylphényl)-1,2,3,6-tétrahydropyridine.
A l'analyse calorimétrique différentielle, la Forme III obtenue dans cette préparation a présenté
· une température de transition solide-solide de 141-142°C
· une enthalpie de transition de 17,6 J/g.

### EXEMPLE 40

Un mélange de 100 g de chlorhydrate de 1-[2-(2-naphtyl)éthyl]-4-(3-trifluorométhylphényl)-1,2,3,6-tétrahydropyridine dans 1 l d'un mélange éthanol/eau 90/10 est chauffé au reflux sous agitation jusqu'à dissolution complète du produit. La solution ainsi obtenue est refroidie de la température de reflux à 5°C sous agitation impeller à 400 tr/minute à une vitesse de refroidissement de 10°C/heure. Le produit cristallin ainsi obtenu est filtré et séché à 45°C sous vide pendant une nuit.
Dans ces conditions, on a obtenu le chlorhydrate de 1-[2-(2-naphtyl)éthyl]-4-(3-trifluorométhylphényl)-1,2,3,6-tétrahydropyridine sous forme de mélange Forme I/Forme III, dans un rapport 65,7/34,3.
A l'analyse calorimétrique différentielle, la Forme I/III obtenue dans cette préparation présente un thermogramme qui montre uniquement les deux pics caractéristiques correspondant aux Formes I et III.

### EXEMPLE 41

Une solution de 3 g de chlorhydrate de 1-[2-(2-naphtyl)éthyl]-4-(3-trifluorométhylphényl)-1,2,3,6-tétrahydropyridine dans 300 ml d'éthanol est atomisée dans un appareil "mini Spray Dryer Buchi" selon le principe de l'atomisation par buse en courant parallèle, en réglant le débit de la pompe, l'aspiration, le chauffage et le flux de courant de façon à avoir une température d'entrée de 172°C, une température de sortie de 107°C et une dépression de 40 mbar. Dans ces conditions, on obtient un produit monopic évasé en DSC avec le maximum à 145°C. Les particules obtenues sont sphériques et la population très homogène ne dépasse pas 5 micromètres de taille moyenne.

### EXEMPLE 42

On introduit dans la chambre de micronisation (diamètre 200 mm) d'un microniseur ALPINE 200 AS 24 kg de chlorhydrate de 1-[2-(2-naphtyl)éthyl]-4-(3-trifluorométhylphényl)-1,2,3,6-tétrahydropyridine - Forme I/III, décrit dans l'Exemple 40, à une vitesse de 25 kg/heure et à une pression de travail de 6,5 bar et on récupère le produit ainsi micronisé dans une manche filtrante. On obtient ainsi un produit micronisé ayant une distribution particulaire selon laquelle la totalité des particules a une taille inférieure à 20 micromètres et 85% des particules ont une taille inférieure à 10 micromètres.

L'analyse calorimétrique différentielle du produit micronisé ainsi obtenu montre que les températures de transition ne sont pas affectées par la micronisation. Lesdites transitions sont du type solide-solide. Le composé se dégrade avant la fusion, qui commence à 250°C.

### EXEMPLE 43

Composition pharmaceutique contenant, en tant que principe actif, le chlorhydrate de 1-[2-(2-naphtyl)éthyl]-4-(3-trifluorométhylphényl)-1,2,3,6-tétrahydropyridine Forme I/III (micronisée) selon l'Exemple 42 ci-dessus:

| | |
|---|---|
| Principe actif | 2,192 mg |
| Amidon de maïs | 141,208 mg |
| Silice colloïdale anhydre | 0,200 mg |
| Stéarate de magnésium | 0,400 mg |
| Cellulose microcristalline | 26,000 mg |

Le principe actif est tamisé à 0,2 mm, puis prémélangé avec les excipients. Ce mélange est tamisé à 0,315 mm, remélangé, puis à nouveau tamisé à 0,315 mm. Après un dernier mélange, on introduit la composition dans des gélules de gélatine n° 3, à raison de 170 mg de composition contenant une quantité de chlorhydrate de 1-[2-(2-naphtyl)éthyl]-4-(3-trifluorométhylphényl)-1,2,3,6-tétrahydropyridine-Forme I/III correspondant à 2 mg de 1-[2-(2-naphtyl)éthyl)-4-(3-trifluorométhylphényl)-1,2,3,6-tétrahydropyridine base.

## Revendications

1. Utilisation d'un composé de formule (I): 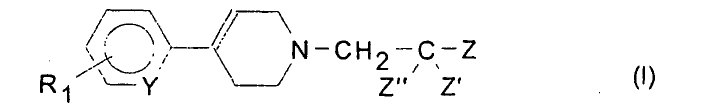 dans laquelle:
- R₁ représente un halogène ou un groupe CF₃, (C₁-C₄)alkyle ou (C₁-C₄)alcoxy;
- Y représente un atome d'azote ou un groupe CH;
- Z' et Z" représentent chacun l'hydrogène ou un groupe (C₁-C₃) alkyle, ou bien l'un représente l'hydrogène et l'autre un groupe hydroxy, ou encore les deux, ensemble, représentent un groupe oxo;
- Z représente
◆ un radical phényle;
◆ un radical phényle monosubstitué par un substituant X, X étant
(a) un groupe (C₁-C₆)alkyle, (C₁-C₆)alcoxy, (C₃-C₇)carboxyalkyle, (C₁-C₄)alcoxycarbonyl(C₁-C₆)alkyle, (C₃-C₇)carboxyalcoxy ou (C₁-C₄)alcoxycarbonyl(C₁-C₆)alcoxy ;
(b) un groupe choisi parmi un (C₃-C₇)cycloalkyle, (C₃-C₇)cycloalkyloxy, (C₃-C₇)cycloalkylméthyle, (C₃-C₇)cycloalkylamino et cyclohexényle, ledit groupe pouvant être substitué par un halogène, hydroxy, (C₁-C₄)alcoxy, carboxy, (C₁-C₄)alcoxycarbonyle, amino, mono- ou di-(C₁-C₄)alkylamino; ou
(c) un groupe choisi parmi un phényle, phénoxy, phénylamino, N-(C₁-C₃)alkylphénylamino, phénylméthyle, phényléthyle, phénylcarbonyle, phénylthio, phénylsulfonyle, phénylsulfinyle ou styryle, ledit groupe pouvant être mono- ou polysubstitué sur le groupe phényle par un halogène, CF₃, (C₁-C₄)alkyle, (C₁-C₄)alcoxy, cyano, amino, mono- ou di-(C₁-C₄)alkylamino, (C₁-C₄)acylamino, carboxy, (C₁-C₄)alcoxycarbonyle, aminocarbonyle, mono- ou di-(C₁-C₄)alkylaminocarbonyle, amino(C₁-C₄)alkyle, hydroxy(C₁-C₄)alkyle ou halogéno(C₁-C₄)alkyle;
◆ un radical phényle disubstitué par un substituant R₂, R₂ étant un halogène ou un groupe hydroxy, méthyle, éthyle, (C₃-C₆)alkyle, (C₁-C₄)alcoxy ou trifluorométhyle et par un substituant X, X étant tel que défini ci-dessus;
◆ un radical 1-naphtyle ou 2-naphtyle;
◆ un radical 1-naphtyle ou 2-naphtyle, substitué dans les positions 5, 6, 7 et/ou 8 par un ou deux groupes hydroxyle, un ou deux groupes (C₁-C₄) alcoxy, ou un groupe 6, 7-méthylènedioxy;
- ou bien Z" est l'hydrogène et Z et Z' représentent, chacun indépendamment, un groupe phényle non substitué ou mono-, di- ou trisubstitué;
ou d'un de ses sels et solvates pharmaceutiquement acceptables, pour la préparation de compositions pharmaceutiques destinées à combattre les maladies entraînant une démyélinisation.

2. Utilisation selon la revendication 1 d'un composé de formule (I) dans laquelle Y est CH, R, est trifluorométhyle, Z' et Z" sont l'hydrogène et Z est tel que défini dans la revendication 1.

3. Utilisation selon la revendication 2 d'un composé de formule (I) où Z représente un groupe 2-naphtyle, 6,7-diméthoxy-2-naphtyle ou 6,7-méthylènedioxy-2-naphtyle.

4. Utilisation selon la revendication 2 d'un composé de formule (I) où Z représente :
- soit un phényle monosubstitué par un substituant X, X étant tel que défini dans la revendication 1 ;
- soit un phényle disubstitué par un substituant R₂, R₂ étant un halogène ou un groupe hydroxy, méthyle, éthyle, (C₃-C₆)alkyle, (C₁-C₄)alcoxy ou trifluorométhyle et par un substituant X tel que défini ci-dessus.

5. Utilisation selon la revendication 4 d'un composé de formule (I) où Z représente un phényle monosubstitué par un groupe X', X' étant un phényle, non substitué ou substitué par 1 à 3 halogène, 1 à 3 CF₃, 1 à 3 (C₁-C₄)alkyle, 1 à 3 (C₁-C₄)alcoxy, 1 à 3 cyano, 1 à 3 amino, 1 à 3 mono- ou di-(C₁-C₄)alkylamino, 1 à 3 (C₁-C₄)acylamino, 1 à 3 carboxy, 1 à 3 (C₁-C₄)alcoxycarbonyle, 1 à 3 aminocarbonyle, 1 à 3 mono- ou di-(C₁-C₄)alkylaminocarbonyle, 1 à 3 amino(C₁-C₄)alkyle, 1 à 3 hydroxy(C₁-C₄)alkyle ou 1 à 3 halogéno(C₁-C₄)alkyle; ou bien un phényle disubstitué par un substituant R₂, R₂ étant tel que défini à la revendication 4 et par un substituant X', X' étant tel que défini ci-dessus.

6. Utilisation selon la revendication 4 d'un composé de formule (I) où Z est un groupe phényle substitué dans les positions 3 et 4 par un groupe (C₁-C₆) alkyle.

7. Utilisation selon la revendication 1 d'un composé de formule (I) dans laquelle Y est CH, R₁ est trifluorométhyle, Z" est l'hydrogène et Z et Z', identiques, représentent chacun un groupe phényle; un groupe phényle substitué en position 2, 3 ou 4 par un atome de fluor, de chlore ou par un groupe méthyle, éthyle, *n*-propyle, *i*-propyle, *n*-butyle, *i*-butyle, *s*-butyle, *t*-butyle trifluorométhyle, cyano, méthoxy, méthylthio, méthylsulfonyle, éthoxy, éthylthio, éthylsulfonyle, (C₁-C₃)alcoxycarbonyle ou di(C₁-C₃)alkylaminocarbonyle; un groupe phényle disubstitué dans les positions 2, 4; 3, 4; 3, 5 ou 2, 6 par un atome de chlore, de fluor, ou par un groupe méthyle, éthyle, trifluorométhyle, cyano ou méthoxy; ou un groupe phényle trisubstitué dans les positions 3, 4, 5; 2, 4, 5 ou 2, 4, 6 par un atome de chlore, de fluor, ou par un groupe méthyle, éthyle, trifluorométhyle, cyano ou méthoxy.

8. Utilisation selon la revendication 1, **caractérisée en ce que** le composé de formule (I) est le chlorhydrate de 1-[2-(2-naphtyl)éthyl]-4-(3-trifluorométhylphényl)-1,2,3,6-tétrahydropyridine.

9. Utilisation selon la revendication 8, **caractérisée en ce que** le chlorhydrate de 1-[2-(2-naphtyl)éthyl]-4-(3-trifluorométhylphényl)-1,2,3,6-tétrahydropyridine est sous forme atomisée ou micronisée.

10. Utilisation selon la revendication 8, **caractérisée en ce que** le chlorhydrate de 1-[2-(2-naphtyl)éthyl]-4-(3-trifluorométhylphényl)-1,2,3,6-tétrahydropyridine est un melange micronisé des formes cristallines I et III dans le rapport d'environ 66/34.

11. Utilisation selon la revendication 1 où le composé de formule (I) est choisi parmi les composés:
- 1-(2-napht-2-yléthyl)-4-(3-trifluorométhylphényl)-1,2,3,6-tétrahydropyridine;
- 1-[2-(6,7-dimétoxynapht-2-yl)éthyl)-4-(3-trifluorométhylphényl)-1,2,3,6-tétrahydropyridine;
- 1-[2-(6,7-méthylènedioxynapht-2-yl)éthyl)-4-(3-trifluorométhylphényl)-1,2,3,6-tétrahydropyridine;
- 1-[2-(4-isobutylphényl)-propyl]-4-(3-trifluorométhylphényl)-1,2,3,6-tétrahydropyridine;
- 1-[(2S)-2-(4-isobutylphényl)propyl]-4-(3-trifluorométhylphényl)-1,2,3,6-tétrahydropyridine;
- 1-[(2R)-2-(4-isobutylphényl)-propyl]-4-(3-trifluorométhylphényl)-1,2,3,6-tétrahydropyridine;
- 1-[2-(4-isobutylphényl)-éthyl]-4-(3-trifluorométhylphényl)-1,2,3,6-tétrahydropyridine;
- 1-[2-(4-tertbutylphényl)-éthyl]-4-(3-trifluorométhylphényl)-1,2,3,6-tétrahydropyridine;
- 1-[2-(4-isobutylphényl)-2-méthylpropyl]-4-(3-trifluorométhylphényl)-1,2,3,6-tétrahydropyridine;
- 1-[2-(4-isopropylphényl)éthyl]-4-(3-trifluorométhylphényl)-1,2,3,6-tétrahydropyridine;
- 1-[2-(3'-chloro-4-biphénylyl)éthyl]-4-(3-trifluorométhylphényl)-1,2,3,6-tétrahydropyridine;
- 1-[2-(2'-chloro-4-biphénylyl)-éthyl]-4-(3-trifluorométhyl-phényl)-1,2,3,6-tétrahydropyridine;
- 1-[2-(4'-chloro-4-biphénylyl)-éthyl]-4-(3-trifluorométhylphényl)-1,2,3,6-tétrahydropyridine;
- 1-[2-(4'-fluoro-4-biphénylyl)-éthyl]-4-(3-trifluorométhylphényl)-1,2,3,6-tétrahydropyridine;
- 1-[2-(3'-trifluorométhyl-4-biphénylyl)-éthyl]-4-(3-trifluorométhylphényl)-1,2,3,6-tétrahydropyridine;
- 1-[2-(4-cyclohexylphényl)éthyl]-4-(3-trifluorométhylphényl)-1,2,3,6-tétrahydropyridine;
- 1-[-2-(4-biphénylyl)-2-éthyl]-4-(4-fluorophényl)-1,2,3,6-tétrahydropyridine;
- 1-[2-(4-biphénylyl)-2-méthylpropyl]-4-(3-trifluorométhylphényl)-1,2,3,6-tétrahydropyridine;
- 1-[2-(4-phénoxyphényl)-2-éthyl]-4-(3-trifluorométhylphényl)-1,2,3,6-tétrahydropyridine;
- 1-[2-(4-benzylphényl)-2-éthyl]-4-(3-trifluorométhylphényl)-1,2,3,6-tétrahydropyridine;
- 1-[2-(4-n-butylphényl)éthyl]-4-(3-trifluorométhylphényl)-1,2,3,6-tétrahydropyridine;
- 1-[2-(4-biphénylyl)éthyl]-4-(3-trifluorométhylphényl)-1,2,3,6-tétrahydropyridine;
- 1-[2-(4-n-butoxyphényl)éthyl]-4-(3-trifluorométhylphényl)-1,2,3,6-tétrahydropyridine;
- 1-[2-(3,4-diéthylphényl)éthyl]-4-(3-trifluorométhylphényl)-1,2,3,6-tétrahydropyridine;
- 1-[2-(3-méthyl-4-pentylphényl)éthyl]-4-(3-trifluorométhylphényl)-1,2,3,6-tétrahydropyridine;
- 1-[2-(4-méthyl-3-pentylphényl)éthyl]-4-(3-trifluorométhylphényl)-1,2,3,6-tétrahydropyridine;
- 1-[2-(3,4-diéthylphényl)éthyl]-4-(6-chloropyrid-2-yl)-1,2,3,6-tétrahydropyridine;
- 1-(2,2-diphényléthyl)-4-(3-trifluorométhylphényl)-1,2,3,6-tétrahydropyridine;
- 1-[2,2-(4,4'-dichlorodiphényl)éthyl]-4-(3-trifluorométhylphényl)-1,2,3,6-tétrahydropyridine;
- 1-[2,2-(3,3'-bistrifluorométhyldiphényl)éthyl]-4-(3-trifluorométhylphényl)-1,2,3,6-tétrahydropyridine;
- 1-[2,2-(4,4'-diméthoxydiphényl)éthyl]-4-(3-triftuorométhylphényl)-1,2,3,6-tétrahydropyridine;
- 1-[2-(4-fluorophényl)-2-phényléthyl]-4-(3-trifluorométhylphényl)-1,2,3,6-tétrahydropyridine;
- 1-(3,3-diphénylpropyl)-4-(3-trifluorométhylphényl)-1,2,3,6-tétrahydropyridine;
- 1-[2,2-(4,4'-dichlorodiphényl)éthyl]-4-(6-chloropyrid-2-yl)-1,2,3,6-tétrahydropyridine;
- 1-[2-(3'-chlorobiphényl-4-yl)-2-oxoéthyl]-4-(3-trifluorométhylphényl)-1,2,3,6-tétrahydropyridine;
- 1-[2-(2'-chlorobiphényl-4-yl)-2-oxoéthyl]-4-(3-trifluorométhylphényl)-1,2,3,6-tétrahydropyridine;
- 1-[2-(4'-chlorobiphényl-4-yl)-2-oxoéthyl]-4-(3-trifluorométhylphényl)-1,2,3,6-tétrahydropyridine;
- 1-[2-(4-isobutylphényl)-2-oxoéthyl]-4-(3-trifluorométhylphényl)-1,2,3,6-tétrahydropyridine;
- 1-[2-(4-benzylphényl)-2-oxoéthyl]-4-(3-trifluorométhylphényl)-1,2,3,6-tétrahydropyridine;
- 1-[2-(4-cyclohexylphényl)-2-oxoéthyl]-4-(3-trifluorométhylphényl)-1,2,3,6-tétrahydropyridine;
- 1-[2-(4'-fluorobiphényl-4-yl)-2-oxoéthyl]-4-(3-trifluorométhylphényl)-1,2,3,6-tétrahydropyridine;
- 1-[2-(4-*n*-butylphényl)-2-oxoéthyl]-4-(3-trifluorométhylphényl)-1,2,3,6-tétrahydropyridine;
- 1-[2-(biphényl-4-yl)-2-oxoéthyl]-4-(3-trifluorométhylphényl)-1,2,3,6-tétrahydropyridine;
- 1-[2-(4-*t*-butylphényl)-2-oxoéthyl]-4-(3-trifluorométhylphényl)-1,2,3,6-tétrahydropyridine;
- 1-[2-(4'-trifluorométhylbiphényl-4-yl)-2-oxoéthyl]-4-(3-trifluorométhylphényl)-1,2,3,6-tétrahydropyridine;
- 1-[2-(2,3'-dichloro-4-biphénylyl)-éthyl]-4-(3-trifluorométhylphényl)-1,2,3,6-tétrahydropyridine;
- 1-[2-(3-chloro-4-biphénylyl)-éthyl]-4-(3-trifluorométhylphényl)-1,2,3,6-tétrahydropyridine;
- 1-[2-(3',5'-dichloro-4-biphénylyl)-éthyl]-4-(3-trifluorométhylphényl)-1,2,3,6-tétrahydropyridine;
- 1-[2-(2',4'-dichloro-4-biphénylyl)-éthyl]-4-(3-trifluorométhylphényl)-1,2,3,6-tétrahydropyridine;
- 1-[2-(2-chloro-4-biphénylyl)-éthyl]-4-(3-trifluorométhylphényl)-1,2,3,6-tétrahydropyridine;
- 1-[2-(3'-chloro-4-biphénylyl)-2-méthylpropyl]-4-(3-trifluorométhylphényl)-1,2,3,6-tétrahydropyridine;
- 1-[2-(2-fluoro-4-biphénylyl)-propyl]-4-(3-trifluorométhylphényl)-1,2,3,6-tétrahydropyridine;
- 1-[2-(4-méthoxy-3-biphénylyl)-éthyl]-4-(3-trifluorométhylphényl)-1,2,3,6-tétrahydropyridine;
- 1-[2-(4'-méthoxy-4-biphénylyl)-éthyl]-4-(3-trifluorométhylphényl)-1,2,3,6-tétrahydropyridine;
- 1-[2-(4'-hydroxy-4-biphénylyl)-éthyl]-4-(3-trifluorométhylphényl)-1,2,3,6-tétrahydropyridine;
- 1-[2-(4'-éthoxycarbonylbutoxy-4-biphénylyl)-éthyl]-4-(3-trifluorométhylphényl)-1,2,3,6-tétrahydropyridine;
- 1-[2-(3-biphénylyl)-éthyl]-4-(3-trifluorométhylphényl)-1,2,3,6-tétrahydropyridine;
- 1-[2-(3'chloro-4'-fluoro-4-biphénylyl)-éthyl]-4-(3-trifluorométhylphényl)-1,2,3,6-tétrahydropyridine;
- 1-[2-(2' trifluorométhyl-4-biphénylyl)-éthyl]-4-(3-trifluorométhylphényl)-1,2,3,6-tétrahydropyridine;
- 1-[2-(3,4-diisobutylphényl)-éthyl]-4-(3-trifluorométhylphényl)-1,2,3,6-tétrahydropyridine;
- 1-[2-(3,4-dipropylphényl)-éthyl]-4-(3-trifluorométhylphényl)-1,2,3,6-tétrahydropyridine;
- 1-[2-(4-cyclohexylphényl)-éthyl]-4-(6-chloropyrid-2-yl)-1,2,3,6-tétrahydropyridine;
- 1-[2-(4-isobutylphényl)-propyl]-4-(6-chloropyrid-2-yl)-1,2,3,6-tétrahydropyridine;
- 1-[2-(2'-trifluorométhylbiphényl-4-yl)-2-oxoéthyl]-4-(3-trifluorométhylphényl)-1,2,3,6-tétrahydropyridine;
- 1-[2-(3'-trifluorométhylbiphényl-4-yl)-2-oxoéthyl]-4-(3-trifluorométhylphényl)-1,2,3,6-tétrahydropyridine;
ou leurs sels ou solvates pharmaceutiquement acceptables.

12. Utilisation selon l'une des revendications 1 à 10 pour la préparation de médicaments destinés au traitement de la sclérose en plaques.

## Patentansprüche

1. Verwendung einer Verbindung der Formel (I): 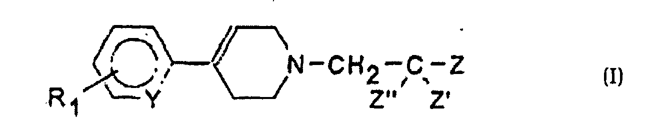 in der:
- R₁ ein Halogen oder eine CF₃-, (C₁-C₄)-Alkyl- oder (C₁-C₄)-Alkoxygruppe bedeutet;
- Y ein Stickstoffatom oder eine Gruppe CH bedeutet;
- Z' und Z" jeweils Wasserstoff oder eine (C₁-C₃)-Alkylgruppe bedeuten oder eine dieser Gruppen Wasserstoff und die andere eine Hydroxygruppe oder beide Gruppen gemeinsam eine Oxogruppe darstellen;
- Z:
◆ eine Phenylgruppe;
◆ eine durch einen Substituenten X monosubstituierte Phenylgruppe, wobei X
(a) eine (C₁-C₆)-Alkyl-, (C₁-C₆)-Alkoxy-, (C₃-C₇)-Carboxyalkyl-, (C₁-C₄)-Alkoxycarbonyl-(C₁-C₆)-alkyl-, (C₃-C₇)-Carboxyalkoxy- oder (C₁-C₄)-Alkoxycarbonyl-(C₁-C₆)-alkoxygruppe;
(b) eine Gruppe ausgewählt aus (C₃-C₇)-Cycloalkyl, (C₃-C₇)-Cycloalkyloxy, (C₃-C₇)-Cycloalkylmethyl, (C₃-C₇)-Cycloalkylamino und Cyclohexenyl, wobei die Gruppe durch ein Halogen, Hydroxy, (C₁-C₄)-Alkoxy, Carboxy, (C₁-C₄)-Alkoxycarbonyl, Amino, Mono- oder Di-(C₁-C₄)-alkylamino substituiert sein kann; oder
(c) eine Gruppe ausgewählt aus Phenyl, Phenoxy, Phenylamino, N-(C₁-C₄)-Alkylphenylamino, Phenylmethyl, Phenylethyl, Phenylcarbonyl, Phenylthio, Phenylsulfonyl, Phenylsulfinyl oder Styryl, wobei die Gruppe an der Phenylgruppe mono- oder polysubstituiert sein kann durch Halogen, CF₃, (C₁-C₄)-Alkyl, (C₁-C₄)-Alkoxy, Cyano, Amino, Mono- oder Di-(C₁-C₄)-alkylamino, (C₁-C₄)-Acylamino, Carboxy, (C₁-C₄)-Alkoxycarbonyl, Aminocarbonyl, Mono- oder Di-(C₁-C₄)-alkylaminocarbonyl, Amino-(C₁-C₄)-alkyl, Hydroxy-(C₁-C₄)-alkyl oder Halogen-(C₁-C₄)-alkyl darstellt;
◆ eine Phenylgruppe, die durch einen Substituenten R₂ disubstituiert ist, wobei R₂ ein Halogen oder eine Hydroxy-, Methyl-, Ethyl-, (C₃-C₆)-Alkyl-, (C₁-C₄)-Alkoxy- oder Trifluormethylgruppe bedeutet, und durch einen Substituenten X, wobei X die oben angegebenen Bedeutungen besitzt;
◆ eine 1-Naphthyl- oder 2-Naphthylgruppe;
◆ eine 1-Naphthyl- oder 2-Naphthylgruppe, die in den Stellungen 5, 6, 7 und/oder 8 durch eine oder zwei Hydroxylgruppen, eine oder zwei (C₁-C₄)-Alkoxygruppen oder eine 6,7-Methylendioxygruppe substituiert ist; bedeutet;
- oder Z" Wasserstoff bedeutet und Z und Z' jeweils unabhängig voneinander eine nichtsubstituierte, mono-, di- oder trisubstituierte Phenylgruppe bedeuten,
oder eines ihrer pharmazeutisch annehmbaren Salze oder Solvate, für die Herstellung von pharmazeutischen Zubereitungen zur Bekämpfung von Erkrankungen, die eine Demyelinisierung zur Folge haben.

2. Verwendung nach Anspruch 1 einer Verbindung der Formel (I), in der Y CH, R₁ Trifluormethyl, Z' und Z" Wasserstoff bedeuten und Z die in Anspruch 1 angegebenen Bedeutungen besitzt.

3. Verwendung nach Anspruch 2 einer Verbindung der Formel (I), worin Z eine 2-Naphthyl-, 6,7-Dimethoxy-2-naphthyl- oder 6,7-Methylendioxy-2-naphthylgruppe bedeutet.

4. Verwendung nach Anspruch 2 einer Verbindung der Formel (I), in der Z:
- entweder eine Phenylgruppe, die durch einen Substituenten X monosubstituiert ist, wobei X die in Anspruch 1 angegebenen Bedeutungen besitzt;
- oder eine Phenylgruppe, die durch einen Substituenten R₂, worin R₂ ein Halogen oder eine Hydroxy-, Methyl-, Ethyl-, (C₃-C₆)-Alkyl-, (C₁-C₄)-Alkoxy- oder Trifluormethylgruppe bedeutet, und einen Substituenten X, wie er oben definiert worden ist, disubstituiert ist, bedeutet.

5. Verwendung nach Anspruch 4 einer Verbindung der Formel (I), in der Z eine Phenylgruppe bedeutet, die durch eine Gruppe X' monosubstituiert ist, wobei X' eine Phenylgruppe darstellt, die nicht substituiert ist oder 1 bis 3 Halogene, 1 bis 3 CF₃-Gruppen, 1 bis 3 (C₁-C₄)-Alkylgruppen, 1 bis 3 (C₁-C₄)-Alkoxygruppen, 1 bis 3 Cyanogruppen, 1 bis 3 Aminogruppen, 1 bis 3 Mono- oder Di-(C₁-C₄)-alkylaminogruppen, 1 bis 3 (C₁-C₄)-Acylaminogruppen, 1 bis 3 Carboxygruppen, 1 bis 3 (C₁-C₄)-Alkoxycarbonylgruppen, 1 bis 3 Aminocarbonylgruppen, 1 bis 3 Mono- oder Di-(C₁-C₄)-alkylaminocarbonylgruppen, 1 bis 3 Amino-(C₁-C₄)-alkylgruppen, 1 bis 3 Hydroxy-(C₁-C₄)-alkylgruppen oder 1 bis 3 Halogen-(C₁-C₄)-alkylgruppen substituiert ist; oder eine Phenylgruppe bedeutet, die durch einen Substituenten R₂, worin R₂ die in Anspruch 1 angegebenen Bedeutungen besitzt, und durch einen Substituenten X', worin X' die oben angegebenen Bedeutungen besitzt, disubstituiert ist, bedeutet.

6. Verwendung nach Anspruch 4 einer Verbindung der Formel (I), worin Z eine Phenylgruppe bedeutet, die in den Positionen 3 und 4 durch eine (C₁-C₆)-Alkylgruppe substituiert ist.

7. Verwendung nach Anspruch 1 einer Verbindung der Formel (I), in der Y CH, R₁ Trifluormethyl, Z" Wasserstoff und Z und Z', die identisch sind, jeweils eine Phenylgruppe, eine Phenylgruppe, die in der Position 2, 3 oder 4 durch ein Fluoratom, ein Chloratom oder eine Methyl-, Ethyl, *n*-Propyl-, *i*-Propyl-, *n*-Butyl-, *i*-Butyl-, *s*-Butyl-, *t*-Butyl-, Trifluormethyl-, Cyano-, Methoxy-, Methylthio-, Methylsulfonyl-, Ethoxy-, Ethylthio-, Ethylsulfonyl-, (C₁-C₃)-Alkoxycarbonyl- oder Di-(C₁-C₃)-alkylaminocarbonyl-gruppe substituiert ist; eine Phenylgruppe, die in den Positionen 2, 4; 3, 4; 3, 5 oder 2, 6 durch ein Chloratom, ein Fluoratom oder eine Methyl-, Ethyl-, Trifluormethyl-, Cyano- oder Methoxy-gruppe disubstituiert ist; oder eine Phenylgruppe, die in den Positionen 3, 4, 5; 2, 4, 5 oder 2, 4, 6 durch ein Chloratom, ein Fluoratom oder eine Methyl-, Ethyl-, Trifluormethyl-, Cyanooder Methoxy-gruppe trisubstituiert ist, bedeuten.

8. Verwendung nach Anspruch 1, **dadurch gekennzeichnet, daß** die Verbindung der Formel (I) 1-[2-(2-Naphthyl)-ethyl]-4-(3-trifluormethyl-phenyl)-1,2,3,6-tetrahydropyridin-Hydrochlorid ist.

9. Verwendung nach Anspruch 8, **dadurch gekennzeichnet, daß** das 1-[2-(2-Naphthyl)-ethyl]-4-(3-trifluormethylphenyl)-1,2,3,6-tetrahydropyridin-Hydrochlorid in zerstäubter oder mikronisierter Form vorliegt.

10. Verwendung nach Anspruch 8, **dadurch gekennzeichnet, daß** das 1-[2-(2-Naphthyl)-ethyl]-4-(3-trifluormethylphenyl)-1,2,3,6-tetrahydropyridin-Hydrochlorid in Form einer mikronisierten Mischung der Kristallformen I und III in einem Verhältnis von etwa 66/34 vorliegt.

11. Verwendung nach Anspruch 1, worin die Verbindung der Formel (I) ausgewählt ist aus den folgenden Verbindungen:
- 1-(2-Naphthyl-2-ylethyl)-4-(3-trifluormethylphenyl)-1,2,3,6-tetrahydropyridin;
- 1-[2-(6,7-Dimethoxynaphth-2-yl)-ethyl)-4-(3-trifluormethylphenyl)-1,2,3,6-tetrahydropyridin;
- 1-[2-(6,7-Methylendioxynaphth-2-yl)-ethyl)-4-(3-trifluormethylphenyl)-1,2,3,6-tetrahydropyridin;
- 1-[2-(4-Isobutylphenyl)-propyl]-4-(3-trifluormethylphenyl)-1,2,3,6-tetrahydropyridin;
- 1-[(2S)-2-(4-Isobutylphenyl)-propyl]-4-(3-trifluormethylphenyl)-1,2,3,6-tetrahydropyridin;
- 1-[(2R)-2-(4-Isobutylphenyl)-propyl]-4-(3-trifluormethylphenyl)-1,2,3,6-tetrahydropyridin;
- 1-[2-(4-Isobutylphenyl)-ethyl]-4-(3-trifluormethylphenyl)-1,2,3,6-tetrahydropyridin;
- 1-[2-(4-tert.-Butylphenyl)-ethyl]-4-(3-trifluormethylphenyl)-1,2,3,6-tetrahydropyridin;
- 1-[2-(4-Isobutylphenyl)-2-methylpropyl]-4-(3-trifluormethylphenyl)-1,2,3,6-tetrahydropyridin;
- 1-[2-(4-Isopropylphenyl)-ethyl]-4-(3-trifluormethylphenyl)-1,2,3,6-tetrahydropyridin;
- 1-[2-(3'-Chlor-4-biphenylyl)-ethyl]-4-(3-trifluormethylphenyl)-1,2,3,6-tetrahydropyridin;
- 1-[2-(2'-Chlor-4-biphenylyl)-ethyl]-4-(3-trifluormethylphenyl)-1,2,3,6-tetrahydropyridin;
- 1-[2-(4'-Chlor-4-biphenylyl)-ethyl]-4-(3-trifluormethylphenyl)-1,2,3,6-tetrahydropyridin;
- 1-[2-(4'-Fluor-4-biphenylyl)-ethyl]-4-(3-trifluormethylphenyl)-1,2,3,6-tetrahydropyridin;
- 1-[2-(3'-Trifluormethyl4-biphenylyl)-ethyl]-4-(3-trifluormethylphenyl)-1,2,3,6-tetrahydropyridin;
- 1-[2-(4-Cyclohexylphenyl)-ethyl]-4-(3-trifluormethylphenyl)-1,2,3,6-tetrahydropyridin;
- 1-[2-(4-Biphenylyl)-2-ethyl]-4-(4-fluorphenyl)-1,2,3,6-tetrahydropyridin;
- 1-[2-(4-Biphenylyl)-2-methylpropyl]-4-(3-trifluormethylphenyl)-1,2,3,6-tetrahydropyridin;
- 1-[2-(4-Phenoxyphenyl)-2-ethyl]-4-(3-trifluormethylphenyl)-1,2,3,6-tetrahydropyridin;
- 1-[2-(4-Benzylphenyl)-2-ethyl]-4-(3-trifluormethylphenyl)-1,2,3,6-tetrahydropyridin;
- 1-[2-(4-n-Butylphenyl)-ethyl]-4-(3-trifluormethylphenyl)-1,2,3,6-tetrahydropyridin;
- 1-[2-(4-Biphenylyl)-ethyl]-4-(3-trifluormethylphenyl)-1,2,3,6-tetrahydropyridin;
- 1-[2-(4-n-Butoxyphenyl)-ethyl]-4-(3-trifluormethylphenyl)-1,2,3,6-tetrahydropyridin;
- 1-[2-(3,4-Diethylphenyl)-ethyl)-4-(3-trifluormethylphenyl)-1,2,3,6-tetrahydropyridin;
- 1-[2-(3-Methyl-4-pentylphenyl)-ethyl]-4-(3-trifluormethylphenyl)-1,2,3,6-tetrahydropyridin;
- 1-[2-(4-Methyl-3-pentylphenyl)-ethyl]-4-(3-trifluormethylphenyl)-1,2,3,6-tetrahydropyridin;
- 1-[2-(3,4-Diethylphenyl)-ethyl]-4-(6-chlorpyrid-2-yl)-1,2,3,6-tetrahydropyridin;
- 1-(2,2-Diphenylethyl)-4-(3-trifluormethylphenyl)-1,2,3,6-tetrahydropyridin;
- 1-[2,2-(4,4'-Dichlordiphenyl)-ethyl]-4-(3-trifluormethylphenyl)-1,2,3,6-tetrahydropyridin;
- 1-[2,2-(3,3'-Bistrifluormethyldiphenyl)-ethyl]-4-(3-trifluormethylphenyl)-1,2,3,6-tetrahydropyridin;
- 1-[2,2-(4,4'-Dimethoxydiphenyl)-ethyl]-4-(3-trifluormethylphenyl)-1,2,3,6-tetrahydropyridin;
- 1-[2-(4-Fluorphenyl)-2-phenylethyl]-4-(3-trifluormethylphenyl)-1,2,3,6-tetrahydropyridin;
- 1-(3,3-Diphenylpropyl)-4-(3-trifluormethylphenyl)-1,2,3,6-tetrahydropyridin;
- 1-[2,2-(4,4'-Dichlordiphenyl)-ethyl]-4-(6-chlorpyrid-2-yl)-1,2,3,6-tetrahydropyridin;
- 1-[2-(3'-Chlorbiphenyl-4-yl)-2-oxoethyl]-4-(3-trifluormethylphenyl)-1,2,3,6-tetrahydropyridin;
- 1-[2-(2'-Chlorbiphenyl-4-yl)-2-oxoethyl]-4-(3-trifluormethylphenyl)-1,2,3,6-tetrahydropyridin;
- 1-[2-(4'-Chlorbiphenyl-4-yl)-2-oxoethyl]-4-(3-trifluormethylphenyl)-1,2,3,6-tetrahydropyridin;
- 1-[2-(4-Isobutylphenyl)-2-oxoethyl]-4-(3-trifluormethylphenyl)-1,2,3,6-tetrahydropyridin;
- 1-[2-(4-Benzylphenyl)-2-oxoethyl]-4-(3-trifluormethylphenyl)-1,2,3,6-tetrahydropyridin;
- 1-[2-(4-Cyclohexylphenyl)-2-oxoethyl]-4-(3-trifluormethylphenyl)-1,2,3,6-tetrahydropyridin;
- 1-[2-(4'-Fluorbiphenyl-4-yl)-2-oxoethyl]-4-(3-trifluormethylphenyl)-1,2,3,6-tetrahydropyridin;
- 1-[2-(4-n-Butylphenyl)-2-oxoethyl]-4-(3-trifluormethylphenyl)-1,2,3,6-tetrahydropyridin;
- 1-[2-(Biphenyl-4-yl)-2-oxoethyl]-4-(3-trifluormethylphenyl)-1,2,3,6-tetrahydropyridin;
- 1-[2-(4-*tert.*-Butylphenyl)-2-oxoethyl]-4-(3-trifluormethylphenyl)-1,2,3,6-tetrahydropyridin;
- 1-[2-(4'-Trifluormethylbiphenyl-4-yl)-2-oxoethyl]-4-(3-trifluormethylphenyl)-1,2,3,6-tetrahydropyridin;
- 1-[2-(2,3'-Dichlor-4-biphenyl)-ethyl]-4-(3-trifluormethylphenyl)-1,2,3,6-tetrahydropyridin;
- 1-[2-(3-Chlor-4-biphenylyl)-ethyl]-4-(3-trifluormethylphenyl)-1,2,3,6-tetrahydropyridin;
- 1-[2-(3',5'-Dichlor-4-biphenylyl)-ethyl]-4-(3-trifluormethylphenyl)-1,2,3,6-tetrahydropyridin;
- 1-[2-(2',4'-Dichlor-4-biphenylyl)-ethyl]-4-(3-trifluormethylphenyl)-1,2,3,6-tetrahydropyridin;
- 1-[2-(2-Chlor-4-biphenylyl)-ethyl]-4-(3-trifluormethylphenyl)-1,2,3,6-tetrahydropyridin;
- 1-[2-(3'-Chlor-4-biphenylyl)-2-methylpropyl]-4-(3-trifluormethylphenyl)-1,2,3,6-tetrahydropyridin;
- 1-[2-(2-Fluor-4-biphenylyl)-propyl]-4-(3-trifluormethylphenyl)-1,2,3,6-tetrahydropyridin;
- 1-[2-(4-Methoxy-3-biphenylyl)-ethyl]4-(3-trifluormethylphenyl)-1,2,3,6-tetrahydropyridin;
- 1-[2-(4'-Methoxy-4-biphenylyl)-ethyl]-4-(3-trifluormethylphenyl)-1,2,3,6-tetrahydropyridin;
- 1-[2-(4'-Hydroxy-4-biphenylyl)-ethyl]-4-(3-trifluormethylphenyl)-1,2,3,6-tetrahydropyridin;
- 1-[2-(4'-Ethoxycarbonylbutoxy-4-biphenylyl)-ethyl]-4-(3-trifluormethylphenyl)-1,2,3,6-tetrahydropyridin;
- 1-[2-(3-Biphenylyl)-ethyl]-4-(3-trifluormethylphenyl)-1,2,3,6-tetrahydropyridin;
- 1-[2-(3'-Chlor-4'-fluor-4-biphenylyl)-ethyl]-4-(3-trifluormethylphenyl)-1,2,3,6-tetrahydropyridin;
- 1-[2-(2'-Trifluormethyl-4-biphenylyl)-ethyl]-4-(3-trifluormethylphenyl)-1,2,3,6-tetrahydropyridin;
- 1-[2-(3,4-Diisobutylphenyl)-ethyl]-4-(3-trifluormethylphenyl)-1,2,3,6-tetrahydropyridin;
- 1-[2-(3,4-Dipropylphenyl)-ethyl]-4-(3-trifluormethylphenyl]-1,2,3,6-tetrahydropyridin;
- 1-[2-(4-Cyclohexylphenyl)-ethyl]-4-(6-chlorpyrid-2-yl)-1,2,3,6-tetrahydropyridin;
- 1-[2-(4-Isobutylphenyl)-propyl]-4-(6-chlorpyrid-2-yl)-1,2,3,6-tetrahydropyridin;
- 1-[2-(2'-Trifluormethylbiphenyl-4-yl)-2-oxoethyl]-4-(3-trifluormethylphenyl)-1,2,3,6-tetrahydropyridin;
- 1-[2-(3'-Trifluormethylbiphenyl-4-yl)-2-oxoethyl]-4-(3-trifluormethylphenyl)-1,2,3,6-tetrahydropyridin;
oder deren pharmazeutisch annehmbare Salze oder Solvate.

12. Verwendung nach einem der Ansprüche 1 bis 10 zur Herstellung von Arzneimitteln, die zur Behandlung der Plättchensklerose bestimmt sind.

## Claims

1. Use of a compound of formula (I): 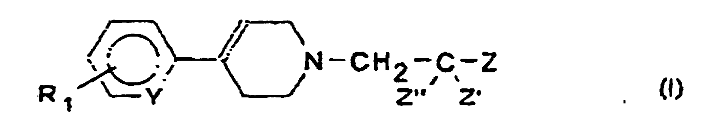 in which:
- R₁ represents a halogen or a CF₃, (C₁-C₄)alkyl or (C₁-C₄)alkoxy group;
- Y represents a nitrogen atom or a CH group;
- Z' and Z" each represent hydrogen or a (C₁-C₃)alkyl group, or alternatively one represents hydrogen and the other a hydroxyl group, or else the two, together, represent an oxo group;
- Z represents
◆ a phenyl radical;
◆ a phenyl radical monosubstituted with a substituent X, X being
(a) a (C₁-C₆)alkyl, (C₁-C₆)alkoxy, (C₃-C₇)carboxyalkyl, (C₁-C₄)alkoxycarbonyl (C₁-C₆) alkyl, (C₃-C₇)carboxyalkoxy or (C₁-C₄)alkoxycarbonyl(C₁-C₆)alkoxy group;
(b) a group chosen from a (C₃-C₇)cycloalkyl, (C₃-C₇)cycloalkyloxy, (C₃-C₇)cycloalkylmethyl, (C₃-C₇)cycloalkylamino and cyclohexenyl, it being possible for the said group to be substituted with a halogen, hydroxyl, (C₁-C₄)alkoxy, carboxyl, (C₁-C₄)alkoxycarbonyl, amino, mono- or di-(C₁-C₄)alkylamino; or
(c) a group chosen from a phenyl, phenoxy, phenylamino, N-(C₁-C₃)alkylphenylamino, phenylmethyl, phenylethyl, phenylcarbonyl, phenylthio, phenylsulphonyl, phenylsulphinyl, or styryl, it being possible for the said group to be mono- or polysubstituted on the phenyl group with a halogen, CF₃, (C₁-C₄)alkyl, (C₁-C₄)alkoxy, cyano, amino, mono- or di(C₁-C₄)-alkylamino, (C₁-C₄)acylamino, carboxyl, (C₁-C₄)alkoxycarbonyl, aminocarbonyl, mono- or di-(C₁-C₄)alkylaminocarbonyl, amino(C₁-C₄)alkyl, hydroxy(C₁-C₄)alkyl or halo(C₁-C₄)alkyl;
◆ a phenyl radical disubstituted with a substituent R₂, R₂ being a halogen or a hydroxyl, methyl, ethyl, (C₃-C₆)alkyl, (C₁-C₄)alkoxy or trifluoromethyl group and with a substituent X, X being as defined above;
◆ a 1-naphthyl or 2-naphthyl radical;
◆ a 1-naphthyl or 2-naphthyl radical substituted at the 5-, 6-, 7- and/or 8-positions with one or two hydroxyl groups, one or two (C₁-C₄)alkoxy groups, or a 6,7-methylenedioxy group;
- or alternatively Z" is hydrogen and Z and Z' represent, each independently, an unsubstituted or a mono-, di- or trisubstituted phenyl group;
or of one of its pharmaceutically acceptable salts and solvates, for the preparation of pharmaceutical compositions intended for combating diseases causing demyelination.

2. Use according to Claim 1, of a compound of formula (I) in which Y is CH, R₁ is trifluoromethyl, Z' and Z" are hydrogen and Z is as defined in Claim 1.

3. Use according to Claim 2, of a compound of formula (I) where Z represents a 2-naphthyl, 6,7-dimethoxy-2-naphthyl, or 6,7-methylenedioxy-2-naphthyl group.

4. Use according to Claim 2, of a compound of formula (I) where Z represents:
- either a phenyl monosubstituted with a substituent X, X being as defined in Claim 1;
- or a phenyl disubstituted with a substituent R₂, R₂ being a halogen or a hydroxyl, methyl, ethyl, (C₃-C₆)alkyl, (C₁-C₄)alkoxy or trifluoromethyl group and with a substituent X as defined above.

5. Use according to Claim 4, of a compound of formula (I) where Z represents a phenyl monosubstituted with a group X', X' being a phenyl, unsubstituted or substituted with 1 to 3 halogen, 1 to 3 CF₃, 1 to 3 (C₁-C₄)alkyl, 1 to 3 (C₁-C₄)alkoxy, 1 to 3 cyano, 1 to 3 amino, 1 to 3 mono- or di-(C₁-C₄)alkylamino, 1 to 3 (C₁-C₄)acylamino, 1 to 3 carboxyl, 1 to 3 (C₁-C₄)alkoxycarbonyl, 1 to 3 aminocarbonyl, 1 to 3 mono- or di-(C₁-C₄)alkylaminocarbonyl, 1 to 3 amino (C₁-C₄)alkyl, 1 to 3 hydroxy(C₁-C₄)alkyl or 1 to 3 halo (C₁-C₄)alkyl; or alternatively a phenyl disubstituted with a substituent R₂, R₂ being as defined in Claim 4 and with a substituent X', X' being as defined above.

6. Use according to Claim 4, of a compound of formula (I) where Z is a phenyl group substituted at the 3- and 4-positions with a (C₁-C₆)alkyl group.

7. Use according to Claim 1, of a compound of formula (I) in which Y is CH, R₁ is trifluoromethyl, Z" is hydrogen and Z and Z', which are identical, each represent a phenyl group; a phenyl group substituted at the 2-, 3- or 4-position with a fluorine or chlorine atom or with a methyl, ethyl, *n*-propyl, *i*-propyl, *n*-butyl, *i*-butyl, *s*-butyl, *t*-butyl, trifluoromethyl, cyano, methoxy, methylthio, methylsulphonyl, ethoxy, ethylthio, ethylsulphonyl, (C₁-C₃)alkoxycarbonyl or di(C₁-C₃)alkylaminocarbonyl group; a phenyl group disubstituted at the 2,4-, 3,4-, 3,5- or 2,6-positions with a chlorine or fluorine atom, or with a methyl, ethyl, trifluoromethyl, cyano or methoxy group; or a phenyl group trisubstituted at the 3,4,5-, 2,4,5- or 2,4,6-positions with a chlorine or fluorine atom, or with a methyl, ethyl, trifluoromethyl, cyano or methoxy group.

8. Use according to Claim 1, **characterized in that** the compound of formula (I) is 1-[2-(2-naphthyl)ethyl)-4-(3-trifluoromethylphenyl)-1,2,3,6-tetrahydropyridine hydrochloride.

9. Use according to Claim 8, **characterized in that** the 1-[2-(2-naphthyl)ethyl)-4-(3-trifluoromethylphenyl)-1,2,3,6-tetrahydropyridine hydrochloride is in atomized or micronized form.

10. Use according to Claim 8, **characterized in that** the 1-[2-(2-naphthyl)ethyl]-4-(3-trifluoromethylphenyl)-1,2,3,6-tetrahydropyridine hydrochloride is a micronized mixture of the crystalline forms I and III in an approximately 66/34 ratio.

11. Use according to Claim 1, where the compound of formula (I) is chosen from the compounds:
- 1-(2-naphth-2-ylethyl)-4-(3-trifluoromethylphenyl)-1,2,3,6-tetrahydropyridine;
- 1-[2-(6,7-dimethoxynaphth-2-yl)ethyl]-4-(3-trifluoromethylphenyl)-1,2,3,6-tetrahydropyridine;
- 1-[2-(6,7-methylenedioxynaphth-2-yl)ethyl]-4-(3-trifluoromethylphenyl)-1,2,3,6-tetrahydropyridine;
- 1-[2-(4-isobutylphenyl)propyl]-4-(3-trifluoromethylphenyl)-1,2,3,6-tetrahydropyridine;
- 1-[(2S)-2-(4-isobutylphenyl)propyl]-4-(3-trifluoromethylphenyl)-1,2,3,6-tetrahydropyridine;
- 1-[(2R)-2-(4-isobutylphenyl)propyl]-4-(3-trifluoromethylphenyl)-1,2,3,6-tetrahydropyridine;
- 1-[2-(4-isobutylphenyl)ethyl]-4-(3-trifluoromethylphenyl)-1,2,3,6-tetrahydropyridine;
- 1-[2-(4-tert-butylphenyl)ethyl]-4-(3-trifluoromethylphenyl)-1,2,3,6-tetrahydropyridine;
- 1-[2-(4-isobutylphenyl)-2-methylpropyl]-4-(3-trifluoromethylphenyl)-1,2,3,6-tetrahydropyridine;
- 1-[2-(4-isopropylphenyl)ethyl]-4-(3-trifluoromethylphenyl)-1,2,3,6-tetrahydropyridine;
- 1-[2-(3'-chloro-4-biphenylyl)ethyl]-4-(3-trifluoromethylphenyl)-1,2,3,6-tetrahydropyridine;
- 1-[2-(2'-chloro-4-biphenylyl)ethyl]-4-(3-trifluoromethylphenyl)-1,2,3,6-tetrahydropyridine;
- 1-[2-(4'-chloro-4-biphenylyl)ethyl]-4-(3-trifluoromethylphenyl)-1,2,3,6-tetrahydropyridine;
- 1-[2-(4'-fluoro-4-biphenylyl)ethyl]-4-(3-trifluoromethylphenyl)-1,2,3,6-tetrahydropyridine;
- 1-[2-(3'-trifluoromethyl-4-biphenylyl)ethyl]-4-(3-trifluoromethylphenyl)-1,2,3,6-tetrahydropyridine;
- 1-[2-(4-cyclohexylphenyl)ethyl]-4-(3-trifluoromethylphenyl)-1,2,3,6-tetrahydropyridine;
- 1-[2-(4-biphenylyl)-2-ethyl]-4-(4-fluorophenyl)-1,2,3,6-tetrahydropyridine;
- 1-[2-(4-biphenylyl)-2-methylpropyl]-4-(3-trifluoromethylphenyl)-1,2,3,6-tetrahydropyridine;
- 1-[2-(4-phenoxyphenyl)-2-ethyl]-4-(3-trifluoromethylphenyl)-1,2,3,6-tetrahydropyridine;
- 1-[2-(4-benzylphenyl)-2-ethyl]-4-(3-trifluoromethylphenyl)-1,2,3,6-tetrahydropyridine;
- 1-[2-(4-n-butylphenyl)ethyl]-4-(3-trifluoromethylphenyl)-1,2,3,6-tetrahydropyridine;
- 1-[2-(4-biphenylyl)ethyl]-4-(3-trifluoromethylphenyl)-1,2,3,6-tetrahydropyridine;
- 1-[2-(4-n-butoxyphenyl)ethyl]-4-(3-trifluoromethylphenyl)-1,2,3,6-tetrahydropyridine;
- 1-[2-(3,4-diethylphenyl)ethyl]-4-(3-trifluoromethylphenyl)-1,2,3,6-tetrahydropyridine;
- 1-[2-(3-methyl-4-pentylphenyl)ethyl]-4-(3-trifluoromethylphenyl)-1,2,3,6-tetrahydropyridine;
- 1-[2-(4-methyl-3-pentylphenyl)ethyl]-4-(3-trifluoromethylphenyl)-1,2,3,6-tetrahydropyridine;
- 1-[2-(3,4-diethylphenyl)ethyl]-4-(6-chloropyrid-2-yl)-1,2,3,6-tetrahydropyridine;
- 1-(2,2-diphenylethyl)-4-(3-trifluoromethylphenyl)-1,2,3,6-tetrahydropyridine;
- 1-[2,2-(4,4'-dichlorodiphenyl)ethyl]-4-(3-trifluoromethylphenyl)-1,2,3,6-tetrahydropyridine;
- 1-[2,2-(3,3'-bistrifluoromethyldiphenyl)ethyl]-4-(3-trifluoromethylphenyl)-1,2,3,6-tetrahydropyridine;
- 1-[2,2-(4,4'-dimethoxydiphenyl)ethyl]-4-(3-trifluoromethylphenyl)-1,2,3,6-tetrahydropyridine;
- 1-[2-(4-fluorophenyl)-2-phenylethyl]-4-(3-trifluoromethylphenyl)-1,2,3,6-tetrahydropyridine;
- 1-(3,3-diphenylpropyl)-4-(3-trifluoromethylphenyl)-1,2,3,6-tetrahydropyridine;
- 1-[2,2-(4,4'-dichlorodiphenyl)ethyl]-4-(6-chloropyrid-2-yl)-1,2,3,6-tetrahydropyridine;
- 1-[2-(3'-chlorobiphenyl-4-yl)-2-oxoethyl]-4-(3-trifluoromethylphenyl)-1,2,3,6-tetrahydropyridine;
- 1-[2-(2'-chlorobiphenyl-4-yl)-2-oxoethyl]-4-(3-trifluoromethylphenyl)-1,2,3,6-tetrahydropyridine;
- 1-[2-(4'-chlorobiphenyl-4-yl)-2-oxoethyl]-4-(3-trifluoromethylphenyl)-1,2,3,6-tetrahydropyridine;
- 1-[2-(4-isobutylphenyl)-2-oxoethyl]-4-(3-trifluoromethylphenyl)-1,2,3,6-tetrahydropyridine;
- 1-[2-(4-benzylphenyl)-2-oxoethyl]-4-(3-trifluoromethylphenyl)-1,2,3,6-tetrahydropyridine;
- 1-[2-(4-cyclohexylphenyl)-2-oxoethyl]-4-(3-trifluoromethylphenyl)-1,2,3,6-tetrahydropyridine;
- 1-[2-(4'-fluorobiphenyl-4-yl)-2-oxoethyl]-4-(3-trifluoromethylphenyl)-1,2,3,6-tetrahydropyridine;
- 1-[2-(4-*n*-butylphenyl)-2-oxoethyl]-4-(3-trifluoromethylphenyl)-1,2,3,6-tetrahydropyridine;
- 1-[2-(biphenyl-4-yl)-2-oxoethyl]-4-(3-trifluoromethylphenyl)-1,2,3,6-tetrahydropyridine;
- 1-[2-(4-*t*-butylphenyl)-2-oxoethyl]-4-(3-trifluoromethylphenyl)-1,2,3,6-tetrahydropyridine;
- 1-[2-(4'-trifluoromethylbiphenyl-4-yl)-2-oxoethyl]-4-(3-trifluoromethylphenyl)-1,2,3,6-tetrahydropyridine;
- 1-[2-(2,3'-dichloro-4-biphenylyl)ethyl]-4-(3-trifluoromethylphenyl)-1,2,3,6-tetrahydropyridine;
- 1-[2-(3-chloro-4-biphenylyl)ethyl]-4-(3-trifluoromethylphenyl)-1,2,3,6-tetrahydropyridine;
- 1-[2-(3',5'-dichloro-4-biphenylyl)ethyl]-4-(3-trifluoromethylphenyl)-1,2,3,6-tetrahydropyridine;
- 1-[2-(2',4'-dichloro-4-biphenylyl)ethyl]-4-(3-trifluoromethylphenyl)-1,2,3,6-tetrahydropyridine;
- 1-[2-(2-chloro-4-biphenylyl)ethyl]-4-(3-trifluoromethylphenyl)-1,2,3,6-tetrahydropyridine;
- 1-[2-(3'-chloro-4-biphenylyl)-2-methylpropyl]-4-(3-trifluoromethylphenyl)-1,2,3,6-tetrahydropyridine;
- 1-[2-(2-fluoro-4-biphenylyl)propyl]-4-(3-trifluoromethylphenyl)-1,2,3,6-tetrahydropyridine;
- 1-[2-(4-methoxy-3-biphenylyl)ethyl]-4-(3-trifluoromethylphenyl)-1,2,3,6-tetrahydropyridine;
- 1-[2-(4'-methoxy-4-biphenylyl)ethyl]-4-(3-trifluoromethylphenyl)-1,2,3,6-tetrahydropyridine;
- 1-[2-(4'-hydroxy-4-biphenylyl)ethyl]-4-(3-trifluoromethylphenyl)-1,2,3,6-tetrahydropyridine;
- 1-[2-(4'-ethoxycarbonylbutoxy-4-biphenylyl)ethyl]-4-(3-trifluoromethylphenyl)-1,2,3,6-tetrahydropyridine;
- 1-[2-(3-biphenylyl)ethyl]-4-(3-trifluoromethylphenyl)-1,2,3,6-tetrahydropyridine;
- 1-[2-(3'-chloro-4'-fluoro-4-biphenylyl)ethyl]-4-(3-trifluoromethylphenyl)-1,2,3,6-tetrahydropyridine;
- 1-[2-(2'-trifluoromethyl-4-biphenylyl)ethyl]-4-(3-trifluoromethylphenyl)-1,2,3,6-tetrahydropyridine;
- 1-[2-(3,9-diisobutylphenyl)ethyl]-4-(3-trifluoromethylphenyl)-1,2,3,6-tetrahydropyridine;
- 1-[2-(3,4-dipropylphenyl)ethyl]-4-(3-trifluoromethylphenyl)-1,2,3,6-tetrahydropyridine;
- 1-[2-(4-cyclohexylphenyl)ethyl]-4-(6-chloropyrid-2-yl)-1,2,3,6-tetrahydropyridine;
- 1-[2-(4-isobutylphenyl)propyl]-4-(6-chloropyrid-2-yl)-1,2,3,6-tetrahydropyridine;
- 1-[2-(2'-trifluoromethylbiphenyl-4yl)-2-oxoethyl]-4-(3-trifluoromethylphenyl)-1,2,3,6-tetrahydropyridine;
- 1-[2-(3'-trifluoromethylbiphenyl-4-yl)-2-oxoethyl]-4-(3-trifluoromethylphenyl)-1,2,3,6-tetrahydropyridine;
or their pharmaceutically acceptable salts or solvates.

12. Use according to one of Claims 1 to 10, for the preparation of medicaments intended for the treatment of multiple sclerosis.
